# EUROPÄISCHE PATENTANMELDUNG

(11) **EP 4 327 847 A1**
(43) Veröffentlichungstag der Anmeldung: **28.02.2024**
(21) Anmeldenummer: 22192171.1
(22) Anmeldetag: 25.08.2022
(51) Int. Cl.: A61M 5/20, A61M 5/31, A61M 5/315

(54) **VERBESSERTER AUTOINJEKTOR**

(71) Anmelder: Ypsomed AG, 3401 Burgdorf (CH)
(72) Erfinder: Tschirren, Markus, 3401 Burgdorf (CH); Schrul, Christian, 3401 Burgdorf (CH); Bernhard, Mario, 3401 Burgdorf (CH); Gruenig, Nicolas, 1784 Courtepin (CH); Allenspach, Marcel, 3401 Burgdorf (CH); Kalbermatter, Gabriel, 3401 Burgdorf (CH)
(74) Vertreter: Meier Obertüfer, Jürg

(57) **Zusammenfassung**

Die Erfindung betrifft einen Autoinjektor umfassend - eine Antriebsvorrichtung (80) aufweisend ein erstes Gehäuseteil (10a) - eine Produktbehälteraufnahmevorrichtung (90) aufweisend ein zweites Gehäuseteil (10b) umfassend einen Spritzenhalter (12) ausgebildet zur Aufnahme einer vorgefüllten Fertigspritze (11 und einem zur Ausschüttung eines Medikaments aus dem Produktbehälter (11c) bewegbaren Stopfens (26), wobei das erste Gehäuseteil (10a) und das zweite Gehäuseteil (10b) koaxial in Fügerichtung verbindbar sind, indem an einem der beiden Gehäuseteile (10a, 10b) ein verbindungsseitiger Endabschnitt (10w) so abgestuft ist, dass dieser in das andere der beiden Gehäuseteile (10a, 10b) eingeschoben werden kann, wobei im Bereich des Endabschnitts (lOw) eine erste Rastverbindung (10u,10v) und eine zweite und dritte Rastverbindung (10f, 10g) vorgesehen sind, welche in Fügerichtung einzeln nacheinander einrastbar sind und wobei gegen die Fügerichtung die erste der Rastverbindungen (10u, 10v) lösbar ist und zumindest die dritte oder letzte Rastverbindung (10f, 10g) verriegelt sowie ein Verfahren für die Endmontage eines Autoinjektors.

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf einen Autoinjektor mit einem Energiespeicher zur Ausschüttung einer vorgegebenen Dosis aus einem einmalig genutzten Produktbehälter.

### HINTERGRUND DER ERFINDUNG

Injektionsgeräte oder Injektionsvorrichtungen zum vereinfachten Verabreichen einer Substanz umfassen unter anderem so genannte Autoinjektoren, welche einen Energiespeicher aufweisen, mit welchem die Ausschüttung automatisch, das heisst ohne extern von einem Anwender zuzuführende oder aufzuwendende Kraft, durchgeführt werden kann. Der Energiespeicher speichert die für eine automatische Substanzabgabe erforderliche Energie vorteilhaft in mechanischer Form. Ein solcher Energiespeicher kann eine Feder sein, welche in einem gespannten Zustand in das Injektionsgerät eingebaut wird und durch Entspannen Energie abgibt. Die Energieabgabe erfolgt an eine Kolbenstange oder ein Druckelement, welches einen Kolben in einen Produktbehälter einschiebt. Der Energiespeicher kann auch vorgesehen sein um den Vorgang des Einstechens einer Injektionsnadel zu automatisieren. Alternativ kann der Einstechvorgang manuell erfolgen, also ausschliesslich durch einen Anwender, ohne hierfür in dem Injektionsgerät gespeicherte Energie zu verwenden.

Das Injektionsgerät kann einen Produktbehälterhalter zur Aufnahme eines Produktbehälters umfassen, wobei in dem Produktbehälterhalter der Produktbehälter radial, axial und vorzugsweise auch drehfest gehalten werden kann. Der Produktbehälterhalter kann mit dem Gehäuse der Injektionsvorrichtung axial- und drehfest verbunden sein, oder bei einem Einstech- und/oder Nadelrückzugsvorgang relativ zum Gehäuse bewegbar sein. Der Produktbehälter kann eine Karpule zur wiederholt lösbaren Verbindung mit Einweg-Injektionsnadeln oder eine Einweg-Fertigspritze mit einer damit unlösbar verbundenen Injektionsnadel sein. Der Produktbehälter weist einen hohlzylindrischen Produktbehälterabschnitt auf, der einen Kolben oder Stopfen verschiebbar lagert. Der Kolben kann mit dem Innenumfang des Produktbehälterabschnitts einen Dichtspalt bilden und mittels Kolbenstange in eine distale Richtung verschoben werden, um über die Injektionsnadel Produkt aus dem Produktbehälter abzugeben.

Das Injektionsgerät kann eine Nadelschutzhülse aufweisen, die nach erfolgter Injektion distal über das distale Ende der Injektionsnadel steht oder relativ zu dem Gehäuse unter Entspannung einer Nadelschutzhülsenfeder in diese Position verschoben wird, um den versehentlichen Zugriff auf die Injektionsnadel zu verhindern und ein Verletzungsrisiko zu verringern. Bei einem Autoinjektor kann die Nadelschutzhülse auch als Auslöseelement zum Auslösen der Produktausschüttung dienen, wobei die Nadelschutzhülse hierzu relativ zu dem Gehäuse in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors zumindest als Sichtschutz dient.

Die Patentanmeldung WO 2016/205963 beschreibt einen beispielhaften Autoinjektor, umfassend ein Gehäuse mit Längsachse und einen axial fest im Gehäuse angeordneten Produktbehälter. Der Autoinjektor umfasst weiter eine in einer Längsrichtung zwischen einer proximalen und einer distalen Position verschiebbare Nadelschutzhülse, welche mit einer Nadelschutzfeder gekoppelt ist. Eine Spiral- oder Triebfeder, in welcher Energie für das automatische Ausschütten vom Produkt gespeichert werden kann, ist über ein erstes Ende mit dem Gehäuse und über ein zweites Ende rotationsfest mit einem koaxial zur Längsachse angeordneten Antriebselement in Form einer rotierenden Gewindestange verbunden. Die Gewindestange greift über ein Gewinde in ein im Gehäuse nicht rotierendes Vortriebsglied in Form einer Vortriebshülse, welche bei einer Verschiebung in distale Richtung den Stopfen des Produktbehälters mit einer annähernd konstanten Ausschüttgeschwindigkeit mitbewegt.

Eine Triebfeder als Antrieb zeichnet sich aus durch hohe Kräfte und ist somit geeignet für einen verzögerungsfreien Start der Ausschüttung auch bei Autoinjektoren mit einer langen Lagerdauer. Eine variable Gewindesteigung der Gewindestange kann eine variable Kennlinie der Triebfeder kompensieren um eine möglichst konstante Ausschüttkraft zu gewährleisten. Dadurch können auch grosse Ausschüttmengen von 5 ml oder mehr innerhalb von maximal 60 s gleichmässig und kontinuierlich ausgeschüttet werden. Bei einer gegenüber gängigen Autoinjektoren verlängerten Ausschüttdauer von mehr als 10 s sollte sich der Anwender jederzeit über den bestimmungsgemässen Fortgang der Injektion vergewissern können, um nicht die Injektion in unbeabsichtigter Weise vorzeitig abzubrechen.

Die Patentanmeldung WO 2012/173554 zeigt eine rotierende Anzeige am proximalen Ende eines Autoinj ektors, welche durch eine Torsionsfeder während der Ausschüttung angetrieben wird. Die Anzeige umfasst mehrere Segmente mit unterschiedlichen Farben zur Signalisierung eines initialen Zustandes vor Beginn der Ausschüttung und eines finalen Zustandes nach erfolgter Ausschüttung. Die Segmente sind durch mindestens ein Fenster am proximalen Ende des Autoinjektors sichtbar, welches in seiner Ausdehnung dem Winkelbereich eines Segments entspricht.

Die Patentanmeldung veröffentlicht als CH 714527A2 zeigt eine Injektionsvorrichtung mit einer Kappe zur Entfernung einer Nadelschutzkappe von einem Produktbehälter und ein Verfahren zum Montieren einer Injektionsvorrichtung, wobei die Kappe ein Eingriffselement umfasst, um beim Entfernen der Kappe von der Injektionsvorrichtung das Entfernen der Nadelschutzkappe von dem Produktbehälter zu bewirken.

Die Patentanmeldung US 2014/0330214 A1 zeigt eine Anzeige in Form einer rotierenden Scheibe und mehreren Fensteröffnungen sowie Wandplättchen am proximalen Ende eines Autoinjektors. Die rotierende Scheibe weist eine abwechselnde erhabene Strukturierung zur visuellen Signalisierung auf und wird durch eine Torsionsfeder während der Ausschüttung angetrieben. Die Wandplättchen im Wechsel mit Fensteröffnungen erlauben eine unterbrochene Sicht auf die rotierende Scheibe bzw. auf die vorbeidrehenden Strukturen.

Die Patentanmeldung veröffentlicht als EP 3241580 A1 zeigt eine verbesserte Schnappverbindung zwischen Karpulenhalter und Gerätekörper. Durch reduzierte radiale Dimensionen der Schnappelemente wird die Deformation des Karpulenhalters bei Endzusammenbau verringert und so die Sicherheit gegen Glasbruch der Karpule erhöht.

Die Patentanmeldung veröffentlicht als WO 2013/076246 A1 zeigt einen Autoinjektor mit einem teleskopartig in einer ersten oder zweiten Position gekoppelten Front- und Rückgehäuse welche Kopplung in der zweiten Position durch den Benutzer geöffnet werden kann um eine neue Spritze einzusetzen.

Die Patentanmeldung veröffentlicht als WO 2013/153011 A1 zeigt einen Karpulenhalter welcher mittels einer Schnappverbindung zu einem Gerätegehäuse verbindbar ist. Die Schnappverbindung weist einen verbesserten Kraftfluss auf indem die Abragungen im Gerätegehäuse in Fenster mit abgerundeten Ecken am Karpulenhalter greifen.

Die Patentanmeldung veröffentlicht als WO 2016/055625 A1 zeigt eine verbesserte Kopplung eines Karpulenhalters an das äussere Gehäuse eines Verabreichungsgeräts. Dabei wird eine erste Schnappverbindung zwischen Karpulenhalter und dem äusseren Gehäuse ergänzt durch eine separate axial versetzte zweite Verbindung zwischen Karpulenhalter und dem äusseren Gehäuse sowie durch eine dritte Verbindung als Verdrehsicherung an einer distal sich erstreckenden Abragung des äusseren Gehäuses.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Unter dem Begriff "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektor im Unterschied zu einem Infusionssystem die Injektionsnadel nicht über einen längeren Zeitraum von mehreren Stunden im Gewebe.

### DARSTELLUNG DER ERFINDUNG

Es ist eine Aufgabe der Erfindung einen verbesserten und dabei kostengünstigeren Autoinjektor der eingangs genannten Art zu schaffen. Die Montage des Autoinjektors bzw. seiner Baugruppen und das Einsetzen eines Produktbehälters anlässlich der Endmontage soll zuverlässiger möglich sein und die Bedienung bzw. Handhabung des Autoinjektors einfacher und sicherer. Die von der Endmontage des Autoinjektors zeitlich und örtlich getrennte Vormontage von Baugruppen bzw. Lagerung und Transport sowie Zuführung derselben soll einfacher und sicherer gewährleistet sein, insbesondere soll die Integrität der Baugruppen während Transport und Zuführung erhalten bleiben. Es ist eine weitere Aufgabe der Erfindung, eine Anzeige für einen Autoinj ektor der eingangs genannten Art zu schaffen, welche dem Anwender ein Andauern oder Fortschreiten einer Ausschüttung bei unterschiedlichsten Griffpositionen optisch anzeigen kann. Die Anzeige soll so ausgestaltet sein, dass auch bei ungünstigen, insbesondere nicht vorgesehenen Griffpositionen einer Hand des Anwenders am Autoinjektor der Anwender das Andauern der Ausschüttung erkennen kann, ohne dass er dazu die Griffposition wechseln muss.

Die Aufgaben werden gelöst durch Vorrichtungen mit den Merkmalen der unabhängigen Ansprüche. Bevorzugte Ausführungsformen der Erfindung sind Gegenstand der abhängigen Ansprüche.

Ein erfindungsgemässer Autoinj ektor umfasst ein einteiliges oder mehrteiliges Gehäuse mit einer Längsachse und eine vorgefüllte Fertigspritze mit einem Produktbehälter und einer daran unlösbar befestigten Injektionsnadel oder Kanüle. Die Fertigspritze ist im Gehäuse axial nicht verschiebbar aufgenommen, wobei eine Spitze der Injektionsnadel um mindestens eine Einstechtiefe in distaler Richtung über ein distales Gehäuseende vorsteht. Der Autoinj ektor umfasst weiter eine zur einmaligen Ausschüttung eines maximalen Inhalts des Produktbehälters vorgespannte Torsionsfeder, ein Antriebselement, ein Vortriebselement, und eine Nadelschutzhülse. Zur Ausschüttung von Flüssigkeit aus dem Produktbehälter durch die Injektionsnadel versetzt die Torsionsfeder das Antriebselement in Rotation um die Längsachse, und das rotierende Antriebselement bewirkt eine Linearbewegung des Vortriebselements zur Verschiebung eines Kolbens im Produktbehälter. Die Nadelschutzhülse wird beim Anpressen des Autoinjektors an eine Injektionsstelle und dem dadurch bewirkten Einstechen der Injektionsnadel in die Injektionsstelle um einen Betätigungshub in proximale Richtung bewegt und startet oder ermöglicht dadurch eine Ausschüttung von Flüssigkeit. Der Betätigungshub der Nadelschutzhülse entspricht dabei mindestens der Einstechtiefe der Injektionsnadel.

Der Autoinjektor umfasst schliesslich eine Anzeige zur Signalisierung des Andauerns einer Ausschüttung, aufweisend ein durch das Antriebselement angetriebenes, um die Längsachse rotierendes Anzeigeelement mit einem optischen Kontrastmuster, und ein Fenster am proximalen Ende des Autoinjektors, durch welches das rotierende Kontrastmuster sichtbar ist. Das Fenster ist um die Längsachse vollständig um 360° umlaufend, also nicht durch einen Rahmen oder Steg parallel zur Längsachse unterbrochen, welcher einen toten Winkel für die Sicht auf das Anzeigeelement erzeugt. Das Fenster ist aus einem transparenten und bevorzugt stabilen oder tragfähigen Material gefertigt. Solange ein noch so kleiner Teil des Fensters sichtbar bleibt, also auch wenn der Anwender ungeschickterweise einen überwiegenden Teil des Fensters mit seiner den Autoinjektor haltenden Hand abdeckt, beispielsweise indem er mit dem Handballen am proximalen Ende abstützt, kann das rotierende Anzeigeelement erkannt werden.

Das Kontrastmuster ist höchstens diskret rotationssymmetrisch um die Längsachse, aber nicht kontinuierlich rotationssymmetrisch, damit eine Rotation des Kontrastmusters auch erkannt werden kann. Das Kontrastmuster umfasst mindestens eine Fläche, Zone oder ein graphisches Element in einem von einer Hintergrundfarbe verschiedenem Farb- oder Grauton bzw. Helligkeit, bevorzugt in Form von sich in Rotationsrichtung wiederholenden graphischen Elementen, wie beispielsweise parallelen Linien, insbesondere parallelen Linien welche zur Längsachse des Autoinjektors geneigt und/oder gekrümmt verlaufen. Das Kontrastmuster kann mindestens eine bis ca. 60, insbesondere 8 bis 16 Linien bzw. Flächen bzw. grafische Elemente aufweisen. Das Kontrastmuster enthält insbesondere eine der physiologisch gut wahrnehmbaren Farben grün oder schwarz.

Das Anzeigeelement kann dazu wie folgt hergestellt werden bzw. beschaffen sein.

Das Kontrastmuster kann im Tampondruckverfahren auf den Grundkörper des Anzeigeelements aufgebracht werden.

Das Kontrastmuster kann als mittels 2-Komponenten Spritzguss in unterschiedlicher Farbe hergestellt werden. Bevorzugt werden die beiden Komponenten so aufgeteilt, dass die 1. Komponente, vorzugsweise aus PBT die funktionellen Elemente, wie beispielsweise die Schnapper beinhaltet. Dadurch werden die funktionalen Elemente immer mit demselben Material und vorzugsweise in gleicher Farbe erzeugt. Die visuellen, ästhetischen Elemente wie das Kontramuster können mit der 2. Komponente, vorzugsweise aus eingefärbtem ABS oder PP erzeugt werden und farblich variieren.

Das Kontrastmuster kann mittels Laserverfahren, vorzugsweise mittels UV-Laser erzeugt werden, indem am Anzeigeelement auf der Sichtfläche lokal Material abgetragen, wodurch eine veränderte insbesondere graue Struktur erzeugt wird. Auch kann durch ein Laserverfahren und geeignete Materialwahl die Oberfläche des Anzeigeelements angeschmolzen werden, wodurch Pigmente sichtbar werden oder der Kuststoff aufgrund seiner Inhaltsstoffe aufschäumt und dadurch die optischen Eigenschaften kontrastierend verändert werden. Alternativ kann mittels Laser oder Erosion auf der zuvor polierten Sichtfläche des Spritzgusswerkzeugs lokal und feinstrukturiert Material abgetragen werden, wodurch Strukturen, insbesondere Dach- oder Würfelstrukturen erzeugt werden können, welche später beim Spritzgiessen auf das Kunststoffteil übertragen werden. Solche Strukturen können eine Tiefe bzw. Erhabenheit von 0,01 bis 0,5 mm aufweisen. Diese ist aus Gründen der Nachhaltigkeit eine vorteilhafte Ausführung, da für das Bauteil weder unterschiedliche Materialien gemischt, noch Farbe auf das Material aufgetragen oder das Material lokal durch eine Laserbeschriftung verändert wird.

Das Kontrastmuster kann auch mittels Durchbrüchen in der Sichtfläche des Anzeigeelements geschaffen werden. Dabei erlauben die Durchbrüche den Blick in das dunkel erscheinende Innere der Autoinjektors, insbesondere auf die metallische spiralförmige Torsionsfeder.

Ein Autoinjektor nach der Erfindung ist geeignet zur Ausschüttung der im Produktbehälter enthaltenen Produktmenge während einer gegenüber bekannten Autoinj ektoren erhöhten Ausschüttdauer von mehr als 10 s, bevorzugt mehr als 20 s oder 30 s. Dadurch ist auch bei Produktmengen von über 2.25 ml und von bevorzugt mindestens 3 ml oder 4 ml eine kontinuierliche oder durchschnittliche Ausschüttrate relativ gering, so dass die injizierte Menge an Medikament laufend vom subkutanen Gewebe aufgenommen oder resorbiert werden kann. Das Einhalten einer minimalen Haltezeit zwischen Ausschüttende und Wegbewegen des Autoinjektors von der Einstichstelle ist daher weniger kritisch. Entsprechend ist keine optische, akustische, oder taktile Signalisierung vonnöten, welche dem Anwender das Ende einer Haltezeit anzeigt.

In einer bevorzugten Ausführungsform ist das rotierende Anzeigeelement direkt oder unmittelbar, insbesondere ohne Getriebe, durch das Antriebselement angetrieben. Das Anzeigeelement rotiert also mit der derselben Drehgeschwindigkeit wie das Antriebselement.

In einer weiter bevorzugten Ausführungsform führt das Anzeigeelement während der Ausschüttung weniger als eine Umdrehung, bevorzugt weniger als eine halbe Umdrehung oder gar weniger als ein Drittel einer Umdrehung pro Sekunde aus. Dadurch wird sichergestellt, dass die Drehbewegung des Kontrastmusters von Auge gut verfolgbar ist und nicht etwa für den Betrachter zu einem unbestimmten rotationssymmetrischen Muster verwischt.

In einer bevorzugten Ausführungsform ist weder am Fenster noch am anschliessenden Gehäuse eine optische Markierung zur Feststellung einer relativen Drehposition des Kontrastmusters vorgesehen. Durch das Anzeigeelement erfolgt keine Anzeige eines Anfangs- oder Endzustands, auch kein Relativangabe zum Fortschritt der Ausschüttung, es dient ausschliesslich zur Signalisierung des Fortschreitens oder Andauerns der Ausschüttung.

In einer bevorzugten Ausführungsform sind das rotierende Anzeigeelement und das Gehäuse oder das Fenster zur Erzeugung eines Ausschüttgeräusches ausgebildet, wodurch dem Anwender zusätzlich das Andauern des Ausschüttvorganges akustisch signalisiert wird. Bevorzugt ist das Ausschüttgeräusch ein kontinuierliches Klickgeräusch, welches durch ein Raster am Fenster oder am Gehäuse und ein darin eingreifendes radial oder axial flexibles Eingriffselement am Anzeigeelement, oder umgekehrt durch ein rotierendes Raster am Anzeigeelement und ein stationäres Eingriffselement erzeugt wird. Auf eine separate optische, akustische, und/oder taktile Signalisierung des Ausschüttendes, beispielsweise durch ein mechanisch oder elektronisch erzeugtes Signal, kann in diesem Fall wie auch bei einer rein optischen Bewegungsanzeige verzichtet werden.

In einer bevorzugten Ausführungsform weist das Fenster einen ersten, zylinderförmigen Bereich parallel zur Längsachse, und einen zweiten, in proximaler Richtung nahtlos an den ersten anschliessenden Bereich mit stetig oder kontinuierlich abnehmendem Durchmesser auf. Der erste Bereich ermöglicht eine Sicht auf das Anzeigeelement aus jeder seitlichen Blickrichtung senkrecht zur Längsachse, und der zweite Bereich ermöglicht eine Sicht auf das Anzeigeelement in distaler Blickrichtung von hinten, beziehungsweise bei senkrecht gehaltenem Autoinjektor von oben. Der zweite Bereich ist in Richtung proximal abgerundet oder einschnürend ausgebildet, und kann in einem dritten Bereich senkrecht zur Längsachse enden. Das Anzeigeelement kann eine dem Verlauf des Fensters folgende konvexe Anzeigefläche aufweisen oder eine kegelmantelförmige Anzeigefläche mit einer mittleren Neigung bezüglich der Längsachse. Bevorzugt ist das Fenster bezüglich der Längsachse rotationssymmetrisch, also mit kreisförmigen Querschnitten in Schnittebenen senkrecht zur Längsachse.

In einer vorteilhaften Variante umfasst der Autoinjektor eine Nadelschutzhülse, welche im Auslieferzustand des Autoinjektors distal über das Gehäuse vorsteht und durch eine Nadelschutzfeder in distale Richtung vorgespannt ist. Bei Anpressen des Autoinjektors an eine Injektionsstelle führt die Nadelschutzhülse eine Betätigungsbewegung in proximale Richtung aus und bei Entfernen des Autoinjektors von der Injektionsstelle eine Nadelschutzbewegung in distaler Richtung, um mit einem hülsenförmigen, bevorzugt rotationssymmetrischen Abschnitt die Injektionsnadel seitlich zu umgeben und radial abzudecken. Die Nadelschutzhülse weist an einem distalen Ende einen ringförmigen Flansch oder Fuss auf als vergrösserte Auflagefläche zum Gewebe um die Injektionsstelle. Der Flansch ist permanent mit dem hülsenförmigen Abschnitt verbunden und bevorzugt einstückig mit diesem ausgebildet. Ein maximaler Durchmesser des Flansches ist grösser als ein maximaler Durchmesser des hülsenförmigen Abschnitts.

Bevorzugt ist der äussere Rand oder die Peripherie des Flansches an die Form einer distalen Öffnung im Gehäuse angepasst. Im eingestochenen Zustand bildet der Flansch somit einen Abschluss des Gehäuses. Weiter bevorzugt ist der Flansch konkav gewölbt ausgebildet, so dass der äussere Rand sich weiter distal befindet als der Übergang zwischen Flansch und hülsenförmigem Abschnitt. Gegenüber einem Flansch mit einer streng ebenen, ringförmigen Auflagefläche mit identischem Aussendurchmesser ist dadurch erstens ein Verkippen des aufgesetzten Autoinjektors weniger wahrscheinlich, und zweitens führt die Konzentration der Druckbelastung auf die von der Einstichstelle am weitesten entfernte Peripherie des Flansches zu weniger Gegendruck im Gewebe und somit zu weniger Schmerz beim Anwender.

Weiter bevorzugt umfasst der Autoinjektor eine Gewindestange als Antriebselement und eine Vortriebshülse mit Innengewinde als Vortriebselement, alternativ eine Antriebshülse mit Innengewinde als Antriebselement und eine Gewindestange als Vortriebselement, wobei das Vortriebselement eine Nut oder einen Nocken als Axialführungselement für eine ausschliesslich lineare Vortriebsbewegung im Gehäuse aufweist. Der Autoinjektor ist bevorzugt dimensioniert zur Aufnahme einer vorgefüllten Fertigspritze umfassend den Produktbehälter und die Injektionsnadel, und aufweisend ein Füllvolumen von mindestens 3 ml, bevorzugt mindestens 5 ml.

Zusammengefasst und in anderen Worten kann ein Autoinjektor nach Aspekten der Erfindung wie folgt ausgeführt sein:
Autoinj ektor umfassend
   - ein Gehäuse mit einer Längsachse L und zur Aufnahme eines Produktbehälters,
   - ein Vortriebsglied und ein rotierendes Antriebselement zur Bewegung des Vortriebsglieds in Längsrichtung und zur automatischen Ausschüttung eines in dem Produktbehälter enthaltenen flüssigen Produkts durch eine Injektionsnadel,
   - eine Anzeige zur Signalisierung des Andauerns einer Ausschüttung, umfassend ein durch das Antriebselement angetriebenes rotierendes Anzeigeelement mit einem optischen Kontrastmuster, und umfassend ein Fenster am proximalen Ende des Autoinj ektors, durch welches das rotierende Kontrastmuster sichtbar ist,
   dadurch gekennzeichnet, dass das Fenster ein um die Längsachse vollständig umlaufendes Fenster ist.
Autoinjektor wobei das rotierende Anzeigeelement direkt durch das Antriebselement angetrieben ist.
Autoinj ektor wobei das Anzeigeelement während einer Ausschüttung weniger als eine, bevorzugt weniger als eine halbe Umdrehung pro Sekunde ausführt.
Autoinjektor wobei keine optische Markierung zur Feststellung einer Drehposition des Kontrastmusters vorgesehen ist.
Autoinj ektor wobei das rotierende Anzeigeelement zur Erzeugung eines Ausschüttgeräusches ausgebildet ist.
Autoinjektor wobei das Fenster einen ersten, zylinderförmigen Bereich parallel zur Längsachse, und einen zweiten, in proximaler Richtung nahtlos an den ersten anschliessenden Bereich mit stetig abnehmendem Durchmesser aufweist.
Autoinj ektor weiter umfassend
   - eine Nadelschutzhülse, welche im Auslieferzustand des Autoinjektors distal über das Gehäuse vorsteht, wobei bei Anpressen des Autoinjektors an eine Injektionsstelle die Nadelschutzhülse eine Betätigungsbewegung in proximale Richtung ausführt und bei Entfernen des Autoinjektors von der Injektionsstelle eine Nadelschutzbewegung in distaler Richtung ausführt, um mit einem hülsenförmigen Abschnitt die Injektionsnadel seitlich zu umgeben,
   wobei die Nadelschutzhülse an einem distalen Ende einen ringförmigen Flansch zum Kontakt mit der Injektionsstelle aufweist, mit einem maximalen Durchmesser, welcher grösser ist als der maximale Durchmesser des hülsenförmigen Abschnitts.
Autoinj ektor wobei ein äusserer Rand des Flanschs an eine distale Öffnung im Gehäuse angepasst ist.
Autoinj ektor wobei der Flansch konkav gewölbt ausgebildet ist.
Autoinjektor wobei das Antriebselement eine Gewindestange ist und das Vortriebselement eine Vortriebshülse mit einem Axialführungselement für eine ausschliesslich lineare Vortriebsbewegung im Gehäuse ist.
Autoinjektor weiter aufweisend eine vorgefüllte Fertigspritze umfassend den Produktbehälter und die Injektionsnadel, und aufweisend ein Füllvolumen von mindestens 3 ml, bevorzugt mindestens 5 ml.

Weitere bevorzugte Ausführungsformen der Erfindung, insbesondere für einen verbesserten Autoinjektor werden nachfolgend beschrieben.

In einer bevorzugten Ausführungsform umfasst ein Autoinjektor
- eine Antriebsvorrichtung aufweisend ein hülsenförmiges erstes Gehäuseteil umfassend eine Antriebsbaugruppe, aufweisend eine Federspule mit Triebfeder, ein Antriebselement insbesondere eine Gewindestange, ein Vortriebselement insbesondere eine Kolbenstange mit innenliegendem Gewinde geeignet zum automatischen Vortrieb des Stopfens
- eine Produktbehälteraufnahmevorrichtung aufweisend ein hülsenförmiges zweites Gehäuseteil definierend eine Längsachse welche sich von distal nach proximal erstreckt umfassend einen Spritzenhalter ausgebildet zur Aufnahme einer vorgefüllten Fertigspritze mit einem zylinderförmigem Produktbehälter welcher sich an seinem distalen Ende über eine Schulter verjüngt mit einer Nadelschutzkappe einer Nadel und einem zur Ausschüttung eines Medikaments aus dem Produktbehälter entlang der Längsachse durch die Antriebsbaugruppe bewegbaren Stopfens, eine Gerätekappe in welcher die Nadelschutzkappe aufnehmbar ist,
wobei das erstes Gehäuseteil und das zweites Gehäuseteil koaxial in einer Fügerichtung verbindbar sind, indem an einem der beiden Gehäuseteile, der verbindungsseitige Endabschnitt so abgestuft ist, dass dieser in das andere der beiden Gehäuseteile eingeschoben werden kann, wobei sich zwischen den beiden Gehäuseteilen, im Bereich des Endabschnitts eine axialwirkende umlaufende Spaltführung ausbildet, wobei im Bereich des Endabschnitts eine erste Rastverbindung, und eine zweite oder mittlere und eine dritte oder letzte, finale Rastverbindung vorgesehen sind, welche in Fügerichtung einzeln nacheinander einrastbar sind und wobei gegen die Fügerichtung die erste der Rastverbindungen zerstörungsfrei lösbar ist und zumindest die dritte oder letzte Rastverbindung verriegelt, insbesondere durch Formschluss. Dadurch sind die Gehäuseteile, in Fügerichtung nacheinander in definierten Positionen bzw. definierten linearen Bewegungsschritten verbindbar bzw. gegen die Fügerichtung lösbar wodurch für Lagerung, Transport und Weiterverarbeitung auf einem Automaten Vorteile entstehen. Die Verriegelung durch die zumindest dritte oder letzte Rastverbindung ist nicht zerstörungsfrei zu öffnen wodurch Bedienungs- und Manipulationssicherheit des Autoinj ektors verbessert wird. Dabei erreicht der Autoinjektor mit verriegelter dritter oder letzter Rastverbindung seine Konfiguration und/oder Dimension für die Auslieferung bzw. den Gebrauch.

Bevorzugt ist der Autoinjektor weitergebildet indem das erstes Gehäuseteil und das zweites Gehäuseteil gegenseitig durch eine bezüglich der Längsachse parallele Linearbewegung in der Fügerichtung verbindbar oder gegen die Fügerichtung lösbar sind. Die, insbesondere alle Einschubbewegungen bzw. die Bewegungen zum Fügen der Gehäuseteile in und Lösen der Gehäuseteile gegen die Fügerichtung sind alliniert und verlaufen insbesondere ausschliesslich in der oder parallel zur Längsachse. Nicht mitgemeint sind komplexere Bewegungen der Gehäuseteile welche beispielsweise durch Bajonett-, Schraub- oder Gewindeverschlüsse bedingt sind. Dadurch ist ein kostengünstiges und präzises Handling der Teile auf Montageautomaten ermöglicht und durch die Verbindbarkeit-Lösbarkeit können die Teile vor einer Endmontage, d.h. vor dem Einsetzen eines Produktbehälters in definierter Position zueinander zusammen gelagert oder transportiert werden. Das Innere des so vormontierten Autoinjektors ist geschützt. Die so verbundenen Teile können als Einheit in einem Baugruppenträger platzsparend und sicher gelagert und transportiert werden. Es können die so verbundenen Teile bzw. Baugruppen von einem Automaten einfach übernommen, für das Einsetzen der Fertigspritze in definierter Lage separiert und danach wieder verbunden und schliesslich endmontiert werden.

Bevorzugt ist der Autoinjektor weitergebildet indem durch die erste Rastverbindung, und die zweite und dritte Rastverbindung, eine erste, zweite und dritte Rastposition des ersten Gehäuseteil relativ zum zweiten Gehäuseteil entlang der Längsachse definiert wird. Dies erlaubt eine vereinfachte Automatisierung und Positionierung auf dem Montageautomaten.

Bevorzugt ist der Autoinjektor weitergebildet indem die erste, zweite und dritte Rastverbindung aus je am ersten und zweiten Gehäuseteil, ausgebildeten Nocken und Nuten im Inneren der Spaltführung gebildet sind. Dadurch wird der Schutz vor unbefugter Öffnung bzw. die Manipulationssicherheit erhöht.

Bevorzugt ist der Autoinjektor weitergebildet indem das eine der Gehäuseteile, mit seiner den verjüngten Endabschnitt einleitenden radialen Stufe am verbindungsseitigen Ende des anderen Gehäuseteils umlaufend vollständig anstossbar ist und so die beiden Gehäuseteile zusammen eine absatzlose Mantelfläche bilden wodurch die Handhabung des Autoinj ektors sicherer wird, wobei die radiale Stufe in Umfangrichtung geradlinig oder stetig gewellt oder unstetig abgesetzt sein. Letzteres verbessert die Verdrehsicherung und kann die rotative Positionierung der Gehäuseteile unterstützen. Zudem kann die radiale Stufe zur Achse rechtwinklig oder geneigt umlaufen. Alternativ kann die am einen Gehäuseteil gebildete Stufe am verbindungsseitigen Ende des anderen Gehäuseteils umlaufend unterbrochen anstossbar sein.

Bevorzugt ist der Autoinjektor weitergebildet indem das eine der Gehäuseteile mit seiner den Endabschnitt einleitenden radialen Stufe am verbindungsseitigen Ende des anderen Gehäuseteils umlaufend anstösst, wenn die dritte oder letzte der Rastverbindungen formschlüssig verriegelt ist. Indem die so gebildete Stossfuge durch die Verriegelung geschlossen bleibt, wird eine bessere Verunreinigungsdichte und Manipulationssicherheit erreicht.

Bevorzugt ist der Autoinjektor weitergebildet indem die beiden Gehäuseteile im Bereich des Endabschnitts Längsführungen aufweisen und/oder einen nicht kreisförmigen Querschnitt der Spaltführung ausbilden. So ergibt sich eine Verdrehsicherung und definierte gegenseitige Montagelage der beiden Gehäuseteile, bevorzugt ist die Spaltführung im Querschnitt nicht kreisförmig zentralsymmetrisch, insbesondere 2-fach bzw. 4-fach zentralsymmetrisch d.h. es ergeben sich mögliche Montagepositionen alle 180° bzw. 90°. Weiter bevorzugt sind axial sich erstreckende Strukturen der Rastverbindungen gegenseitig an beiden Gehäuseteilen bezüglich der Längsachse des Autoinj ektors alliniert und bilden so Längsführungen.

Bevorzugt ist der Autoinjektor weitergebildet indem das eine der Gehäuseteile an seinem Endabschnitt zumindest eine Montageöffnung aufweist, welche zumindest in der ersten Rastposition von Aussen zugänglich ist und in zumindest der dritten oder letzten Rastposition vom andern der Gehäuseteile vollständig überdeckt wird. Dadurch kann ein Montagewerkzeug zur Endmontage vor dem finalen Fügen eingesetzt und entfernt werden und die dazu nötige Öffnung wird anschliessend vollständig verschlossen und ist von Aussen nicht mehr sichtbar. Das in das Innere des Gehäuseteils reichende Montagewerkzeug kann dazu dienen, ein im Innern des Gehäuses beweglich gelagertes und/oder kraft- oder drehmomentbeaufschlagte Bauteil während einer Montagebewegung an einem definierten Ort festzuhalten und danach wieder freizugeben. Beispielsweise kann so eine Verschnappung oder Verankerung des Bauteils sichergestellt werden.

Bevorzugt ist der Autoinjektor weitergebildet indem die beiden Gehäuseteile genau drei Rastpositionen entlang der Längsachse einnehmen können.

Bevorzugt ist der Autoinjektor weitergebildet indem die beiden Gehäuseteile in der dritten oder letzten Rastposition alternativ oder zusätzlich zur Rastverbindung durch eine Klebung oder Schweissung gegenseitig befestigt werden. Dies erhöht die Stabilität und Präzision der Verbindung und gewährleistet die Dichte der Stossfuge zwischen den beiden Gehäuseteilen.

Bevorzugt ist der Autoinjektor weitergebildet indem dieser weiter einen Spritzenadapter aufweist, welcher mit seinem distalen Ende an das proximale Ende der Fertigspritze anstossbar ist, wobei das proximale Ende des Spritzenadapters durch ein elastisches Element gebildet ist, welches mit dem ersten Gehäuseteil wirkverbunden ist. Wodurch die Spritze spielfrei in distaler Richtung kraftbeaufschlagt im Spritzenhalter gehalten ist, was die Glasbruchgefahr reduziert.

Bevorzugt ist der Autoinjektor weitergebildet indem das elastische Elemente des Spritzenadapters, an das die Auflagefläche angebunden ist, durch den zweiten Fügeschritt bzw. durch die Bewegung in die dritte oder letzte Rastposition vorgespannt oder weitergespannt wird. Wodurch der Endmontagevorgang vereinfacht wird.

Bevorzugt ist der Autoinjektor weitergebildet indem das Vortriebselement in eine gewünschte Startposition auf das Antriebselement schraubbar ist. So lässt sich im endmontierten Autoinjektor das freie Spiel zwischen Kolben in der Fertigspritze und Vortriebselement aufheben und/oder die Startposition des Vortriebselements an eine Kolbenposition einer teilgefüllte Fertigspritze anpassen.

Zudem umfasst die dargestellte Erfindung ein Verfahren für die Endmontage eines Autoinj ektors welcher eine Längsachse definiert, aufweisend die Schritte:
- Bereitstellen einer Produktbehälteraufnahmevorrichtung aufweisend ein erstes Gehäuseteil
- Bereitstellen einer Antriebsvorrichtung aufweisend ein zweites Gehäuseteil
- Fügen der Gehäuseteile in Fügerichtung in eine durch eine erste Rastverbindung definierte erste Rastposition
- Einsetzen, insbesondere seitliches Einsetzen eines Montagewerkzeugs durch eine Montageöffnung an einem der Gehäuseteile wenn diese in der ersten Rastposition sind
- Fügen der Gehäuseteile in Fügerichtung in eine zweite Rastposition welche durch eine zweite Rastverbindung der Gehäuseteile gehalten wird
- Entfernen des Montagewerkzeugs aus der Montageöffnung an einem der Gehäuseteile, wenn diese in der zweiten Rastposition sind
- Fügen der Gehäuseteile in Fügerichtung in eine dritte Rastposition welche durch eine dritte Rastverbindung der Gehäuseteile gehalten und verriegelt wird
- Vollständiges Abdecken der Montageöffnung an einem der Gehäuseteile durch das andere der Gehäuseteile wenn diese in der dritten oder letzten Rastposition sind.

Das Verfahren für die Endmontage eines Autoinjektors kann bevorzugt weiter einen oder mehrere der folgenden Schritte aufweisen:
- Einstellen bzw. Vorbereiten einer axialen Startposition für ein Vortriebselement durch Aufschrauben auf ein Antriebselement. So lässt sich im endmontierten Autoinjektor das freie Spiel zwischen Kolben in der Fertigspritze und Vortriebselement aufheben und/oder die Startposition des Vortriebselements an eine Kolbenposition einer teilgefüllte Fertigspritze variabel anpassen.
- Positionieren der Produktbehälteraufnahmevorrichtung und der Antriebsvorrichtung koaxial der Längsachse und Fügen der Gehäuseteile in Fügerichtung in eine Rastposition welche durch eine in und gegen die Fügerichtung lösbare Rastverbindung der Gehäuseteile gehalten wird

- Lagern und/oder Transportieren der Produktbehälteraufnahmevorrichtung und der Antriebsvorrichtung zusammen verbunden in der Rastposition der Gehäuseteile
- Bereitstellen einer Fertigspritze
- Lösen der Rastverbindung der Gehäuseteile gegen die Fügerichtung und Separieren der Antriebsvorrichtung von der Produktbehälteraufnahmevorrichtung
- Einsetzen der Fertigspritze koaxial oder achsparallel zur Längsachse in die Produktbehälteraufnahmevorrichtung
- Halten und/oder Positionieren eines im Innern des einen Gehäuseteile beweglichen Bauteils (mittels dem durch die Montageöffnung eingesetzten Montagewerkzeug.
- Spannen eines elastischen Spritzenadapters bei Fügen der Gehäuseteile von der zweiten in die dritte oder letzte Rastposition.

Alternativ oder ergänzend kann das Verfahren für die Endmontage eines Autoinj ektors welcher eine Längsachse definiert mehrere der folgenden Schritte aufweisen:
- Bereitstellen einer Produktbehälteraufnahmevorrichtung aufweisend ein erstes Gehäuseteil
- Bereitstellen einer Antriebsvorrichtung aufweisend ein zweites Gehäuseteil
- Positionieren der Produktbehälteraufnahmevorrichtung und der Antriebsvorrichtung koaxial der Längsachse und Fügen der Gehäuseteile durch eine Linearbewegung in eine erste Rastposition welche durch eine erste in und gegen die Fügerichtung zerstörungsfrei lösbare Rastverbindung der Gehäuseteile gehalten wird
- Lagern und/oder Transportieren der Produktbehälteraufnahmevorrichtung und der Antriebsvorrichtung zusammen verbunden in der ersten Rastposition der Gehäuseteile
- Bereitstellen einer Fertigspritze
- Lösen der Rastverbindung der Gehäuseteile und Separieren der Antriebsvorrichtung von der Produktbehälteraufnahmevorrichtung
- Einsetzen der Fertigspritze koaxial oder achsparallel zur Längsachse in die Produktbehälteraufnahmevorrichtung
- Fügen der Gehäuseteile durch eine Linearbewegung entlang der Längsachse in die erste Rastposition
- Fügen der Gehäuseteile durch eine Linearbewegung entlang der Längsachse in eine zweite Rastposition welche durch eine zweite Rastverbindung der Gehäuseteile gehalten wird
- Fügen der Gehäuseteile durch eine Linearbewegung entlang der Längsachse in eine dritte Rastposition welche durch eine dritte Rastverbindung der Gehäuseteile gehalten und verriegelt wird.

Bevorzugt ist das Verfahren für die Endmontage eines Autoinjektors weitergebildet indem es weiter einen oder mehrere der folgenden Schritte aufweist:
- Seitliches Einsetzen eines Montagewerkzeugs durch eine Montageöffnung an einem der Gehäuseteile wenn diese in der ersten Rastposition sind
- Halten und/oder Positionieren eines im Innern des einen Gehäuseteile beweglichen Bauteils mittels dem durch die Montageöffnung eingesetzten Montagewerkzeug
- Entfernen des Montagewerkzeugs aus der Montageöffnung an einem der Gehäuseteile wenn diese in der zweiten Rastposition sind
- Vollständiges Abdecken der Montageöffnung durch das andere der Gehäuseteile wenn diese in der dritten oder letzten Rastposition sind.

Bevorzugt ist das Verfahren für die Endmontage eines Autoinjektors weitergebildet indem es weiter den folgenden Schritt aufweist:
- Spannen eines elastischen Spritzenadapters beim Fügen bzw. durch die Fügebewegung der Gehäuseteile von der zweiten in die dritte oder letzte Rastposition. Der Spritzenadapter ist dabei so angeordnet, dass die elastischen Elemente die Fügebewegung zumindest teilweise mitmachen.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden. Es zeigen
Fig. 1 die Komponenten eines Autoinjektors,
Fig. 2 zwei Längsschnitte durch den Autoinjektor aus Fig.1 vor der Injektion,
Fig. 3 zwei Ansichten des Anzeigeelements,
Fig. 4 eine Ansicht des distalen Endes der Nadelschutzhülse,
Fig. 5 zwei um 90° gegeneinander gedrehte Ansichten einer zweiten Ausführungsform eines Autoinjektors,
Fig. 6 die Komponenten des Autoinjektors bzw. der Baugruppen aus Fig.5,
Fig. 7 zwei um 90° gegeneinander gedrehte Längsschnitte des Autoinjektors aus Fig. 5 im Zustand Auslieferung,
Fig. 8 zwei um 90° gegeneinander gedrehte Längsschnitte des Autoinjektors aus Fig. 5 im Zustand Auslösesperre,
Fig. 9 zwei um 90° gegeneinander gedrehte Längsschnitte des Autoinjektors aus Fig.5 im Zustand Gerätekappe inkl. Nadelschutzkappe abgezogen,
Fig. 10 zwei um 90° gegeneinander gedrehte Längsschnitte des Autoinjektors aus Fig. 5 im Zustand Eingestochen,
Fig. 11a zwei um 90° gegeneinander gedrehte Längsschnitte des Autoinjektors aus Fig. 5 im Zustand Freigabe Antrieb,
Fig. 11b Ansicht im Teilschnitt der Kupplungshülse und der Federspule des Autoinjektors aus Fig. 5 im Zustand Freigabe Antrieb,
Fig. 12a zwei um 90° gegeneinander gedrehte Längsschnitte des Autoinjektors aus Fig. 5 im Zustand Ausschüttung,
Fig. 12b Ansicht aus proximaler Sicht der Federspule mit der Spiralfeder des Autoinjektors aus Fig. 5,
Fig. 12c Querschnitt aus distaler Sicht des Anzeigeelements mit Anzeigefensters des Autoinjektors aus Fig. 5,
Fig. 13 zwei um 90° gegeneinander gedrehte Längsschnitte des Autoinjektors aus Fig. 5 im Zustand Ausschüttung beendet,
Fig. 14 zwei um 90° gegeneinander gedrehte Längsschnitte des Autoinjektors aus Fig. 5 im Zustand Nadelschutzverriegelung aktiviert,
Fig. 15a zwei um 90° gegeneinander gedrehte Längsschnitte des Autoinjektors aus Fig. 5 im Zustand Ausschüttung abgebrochen,
Fig. 15b Ansicht im Teilschnitt der Kupplungshülse und der Federspule des Autoinjektors aus Fig. 5 im Zustand Ausschüttung abgebrochen,
Fig. 16 eine Ansicht und einen Längsschnitt des Autoinjektors aus Fig. 5 im Zustand Vorverschnappt,
Fig. 17 zwei um 90° gegeneinander gedrehte Längsschnitte der Produktbehälteraufnahmevorrichtung mit Insert vormontiert,
Fig. 18 zwei um 90° gegeneinander gedrehte Längsschnitte der Produktbehälteraufnahmevorrichtung mit Fertigspritze vormontiert,
Fig. 19 zwei um 90° gegeneinander gedrehte Längsschnitte der Produktbehälteraufnahmevorrichtung mit Insert und Fertigspritze endmontiert,
Fig. 20a einen Längsschnitt des Autoinjektors aus Fig. 5 nach dem ersten Fügeschritt,
Fig. 20b einen Längsschnitt des Autoinjektors aus Fig. 5 nach dem zweiten finalen Fügeschritt,
Fig. 21a zeigt einen Längsschnitt der Produktbehälteraufnahmevorrichtung für eine beispielhafte Einstechtiefe von 8 mm im Zustand Auslieferung,
Fig. 21b zeigt einen Längsschnitt der Produktbehälteraufnahmevorrichtung für eine beispielhafte Einstechtiefe von 8 mm im Zustand Eingestochen,
Fig. 22a zeigt einen Längsschnitt durch das distale Ende der Produktbehälteraufnahmevorrichtung für eine beispielhafte Einstechtiefe von 5 mm im Zustand Auslieferung,
Fig. 22b zeigt einen Längsschnitt durch das distale Ende der Produktbehälteraufnahmevorrichtung für eine beispielhafte Einstechtiefe von 5 mm im Zustand Eingestochen,
Fig. 23a zeigt einen Längsschnitt der Antriebsvorrichtung sowie je eine Ansicht der Triebfeder und des Anzeigefensters,
Fig. 23b zeigt einen Längsschnitt durch das proximale Ende der Antriebsvorrichtung sowie je eine Ansicht einer Variante der Triebfeder und des Anzeigefensters,
Fig. 24a zeigt einen Längsschnitt des Autoinjektors aus Fig. 5 mit voll gefüllter Fertigspritze,
Fig. 24b zeigt einen Längsschnitt des Autoinjektors aus Fig. 5 mit teilweise gefüllter Fertigspritze,
Fig. 25 zeigt zwei Ansichten einer Variante des Autoinjektors aus Fig. 5 mit einer Abzugshülse

### FIGURENBESCHREIBUNG

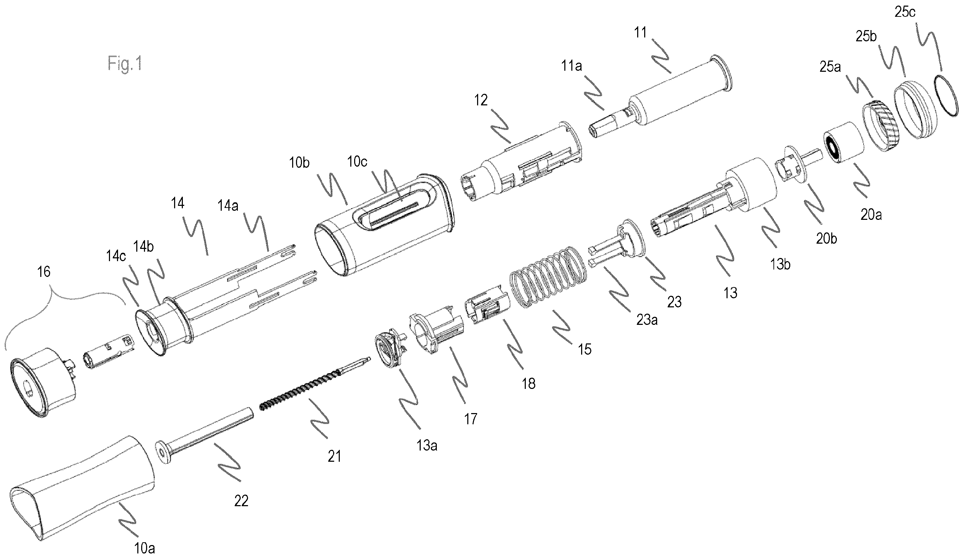

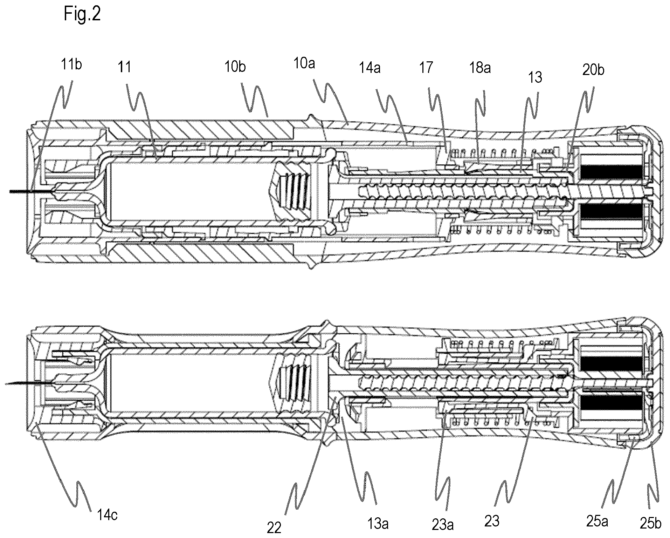

Fig. 1 ist eine Explosionsdarstellung der Komponenten eines Autoinjektors nach der Erfindung in einer ersten Ausführungsform, und Fig. 2 zeigt zwei zueinander um die Längsachse um 90° gedrehte Längsschnitte durch den Autoinjektor nach Fig.1 im eingestochenen Zustand und bereit zur Ausschüttung.

Der Autoinjektor 1 weist ein hülsenförmiges, längliches Gehäuse auf mit einer Längsachse L und umfassend ein distales Gehäuseteil 10b und ein damit unlösbar verschnapptes proximales Gehäuseteil 10a in Form eines Griffs. Ein Produktbehälter in Form einer Fertigspritze 11 mit einer am Produktbehälter unlösbar befestigten Injektionsnadel ist in einem Spritzenhalter 12 gehalten, wobei der Spritzenhalter im Gehäuse axial- und drehfest aufgenommen ist. Die Fertigspritze 11 wird von einem fest im Gehäuseteil 10a verankerten Haltefederabschnitt 13a in distale Richtung in einen Eingriff mit einer Schulter des Spritzenhalters 12 gedrückt. Die Fertigspritze 11 ist in Bezug auf das Gehäuseteil 10b so angeordnet, dass die Spitze der Injektionsnadel um eine der subkutanen oder intramuskulären Einstechtiefe entsprechende Länge über das distale Ende einer proximalen Zwischenposition der Nadelschutzhülse 14 hinausragt und durch eine Nadelschutzhülse 14 vor und nach der Injektion zumindest seitlich geschützt oder abgedeckt wird. Die Nadelschutzhülse 14 wird beim Einstechen der Injektionsnadel in die Injektionsstelle entlang der Längsachse L um einen Betätigungshub und gegen die Kraft einer Nadelschutzfeder 15 in proximale Richtung geschoben und löst dadurch eine Produktausschüttung aus. Die Nadelschutzhülse umfasst dazu zwei Hülsenarme 14a, welche gegenüber zwei als Sichtfenster bezeichneten Ausnehmungen 10c des Gehäuses um 90° um die Längsachse L versetzt beziehungsweise verdreht angeordnet sind. Nach der erfolgten Injektion kann die Nadelschutzhülse 14 relativ zu dem Gehäuseteil 10b aus der betätigten Position entlang der Längsachse L in die distale Richtung in eine Nadelschutzposition verschoben und dort gegen erneutes Zurückschieben blockiert werden. Die Nadelschutzhülse umfasst einen hülsenförmigen oder hohlzylindrischen Abschnitt 14b und einen Flansch 14c am distalen Ende. Der Querschnitt des hohlzylindrischen Abschnitts 14b ist oval, genauso wie der äussere Umfang des Flansches, so dass vorliegend der Flansch eine konstante Breite oder radiale Ausdehnung aufweist.

Ein Federpaket umfasst eine Spiralfeder 20a und eine Federspule 20b. Die Spiralfeder 20a ist mit ihrem äusseren Ende drehfest an einer Federhülse 13b als Teil eines fest im Gehäuse verankerten Mechanikhalters 13 befestigt. Das innere Ende der Spiralfeder 20a ist drehfest mit der Federspule 20b verbunden. Die Federspule 20b umfasst einen Federschaft und einen distalen Federflansch. Die Spiralfeder 20a beziehungsweise die Federspule 20b versetzt ein Antriebselement 21 in eine Rotationsbewegung und ein Vortriebselement 22 in eine bevorzugt rein axiale Vortriebsbewegung. Dazu greift ein Gewindeelement in ein sich über den Ausschütthub erstreckendes Gewinde mit einer variablen Gewindesteigung.

Die Fertigspritze 11 umfasst einen zylindrischen Spritzenkörper als Produktbehälter, an dessen distalen Ende eine hohle Injektionsnadel mit einer Spritzenschulter fest verbunden ist. Die Injektionsnadel der Fertigspritze ist von einer Nadelschutzkappe 11a abgedeckt, welche als sogenanntes Rigid Needle Shield (RNS) ausgebildet ist und ein gummielastisches Nadelschutzelement und eine Hülle aus Hartkunststoff umfasst. Die Nadelschutzkappe schützt die Injektionsnadel gegen mechanische Einwirkungen und Verschmutzung, und hält die Injektionsnadel und das Produkt steril. An dem distalen Ende des Autoinjektors ist in seinem Auslieferungszustand eine zweiteilige Geräte- oder Abziehkappe 16 angeordnet, die vor der Verwendung des Autoinjektors zusammen mit der Nadelschutzkappe 11a axial abgezogen und/oder abgedreht und vollständig entfernt wird.

Eine Schalthülse 17 ist formschlüssig mit einem proximalen Ende der Hülsenarme 14a der Nadelschutzhülse 14 und mit einem distalen Ende der Nadelschutzfeder 15 angeordnet und zumindest teilweise von Letzterer umgeben. Die Schalthülse 17 ist bevorzugt mit dem proximalen Ende der Hülsenarme der Nadelschutzhülse 14 verschnappt. Die Bewegung der Schalthülse 17 in distale Richtung ist durch den Haltefederabschnitt 13a begrenzt, welcher seinerseits nach der Montage der Schalthülse 17 mit dem Mechanikhalter 13 verschnappt. Innerhalb und koaxial zur Schalthülse 17 ist eine Sperrhülse 18 angeordnet, welche über ein sägezahnförmiges, an einem in distale Richtung weisenden Arm federnd angebrachtes Verriegelungsglied 18a derart an die Schalthülse 17 gekoppelt ist, dass eine Betätigungsbewegung von Nadelschutzhülse 14 und Schalthülse 17 auch die Sperrhülse 18 nach proximal bewegt. Durch einen zusätzlichen proximalen Arretierhub der Sperrhülse 18 relativ zur Schalthülse 17 in eine proximale Endposition wird das Verriegelungsglied 18a von der Schalthülse 17 für eine Bewegung nach innen sicher freigegeben. Durch die Federwirkung des Arms hintergreift das Verriegelungsglied 18a eine nach proximal gerichtete Kante des Autoinjektors oder rastet in eine axialfeste Ausnehmung des Autoinjektors ein und arretiert so die Sperrhülse 18 gegen eine distale Bewegung. Beim Entfernen des Autoinjektors von der Einstichstelle wird die Schalthülse 17 durch die Nadelschutzfeder 15 in distaler Richtung über das Verriegelungsglied 18a geschoben, worauf das Verriegelungsglied durch die Federwirkung des Arms in einer Verriegelungsposition eine nach proximal gerichtete Kante der Schalthülse 17 hintergreift und die Schalthülse sowie die Nadelschutzhülse gegen eine erneute Bewegung in proximale Richtung verriegelt oder blockiert.

Eine Kupplungshülse 23 mit zwei Haltenocken 23a ist über Kopplungselemente mit der Federspule 20b gekoppelt. Vor der Ausschüttung greifen die Haltenocken 23a in Ausnehmungen des axialfesten Mechanikhalters 13 ein und sind durch einen Innenumfang der Sperrhülse 18 an einer Bewegung nach aussen gehindert, wodurch sich die Kupplungshülse 23 auch axial nicht bewegen kann. Beim Auslösen der Ausschüttung wird die Sperrhülse 18 durch eine proximale Bewegung der Nadelschutzhülse 14 von der Position der Ausnehmungen wegbewegt, so dass sich die Haltenocken 23a radial lösen können und die Kupplungshülse 23 freigeben wird. Letztere bewegt sich in proximaler Richtung und gibt die Federspule 20b zur Rotation frei, wie ausführlich beschrieben in der Patentanmeldung PCTEP2021076923. Am proximalen Ende des Autoinjektors befindet sich die Anzeige mit einem rotierenden Anzeigeelement 25a mit einem Kontrastmuster in Form von parallelen Streifen, einem umlaufenden transparenten Fenster 25b, und einem proximalen, nichttransparenten Abschluss 25c.

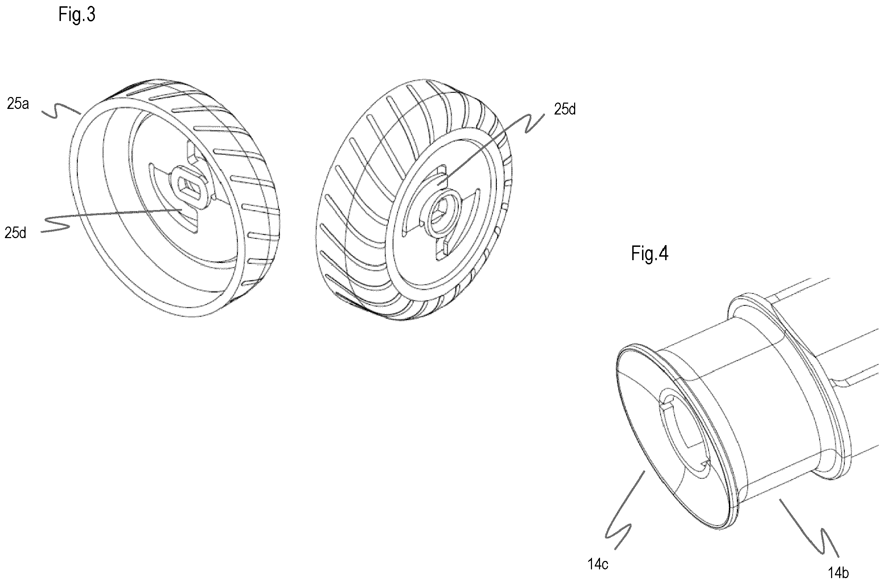

Fig. 3 zeigt zwei Ansichten des Anzeigeelements 25a mit einem Eingriffselement 25d zum Eingriff in ein nichtrotierendes Raster an der Innenseite des Fensters 25b oder des Abschlusses 25d. Eingriffselement 25d ist federnd gelagert und erzeugt bei Rotation des Anzeigeelements während der Ausschüttung eine der Rasterung entsprechende Anzahl von Klickgeräuschen. Das Anzeigeelement hat eine Form welche derjenigen des Fensters folgt beziehungsweise an diese angepasst ist, mit einem ersten, distalen Bereich und einem in proximaler Richtung daran anschliessenden zweiten Bereich. Der erste Bereich ist zylinderförmig, parallel zur Längsachse, während der zweite Bereich einen stetig abnehmenden Durchmesser aufweist.

Fig. 4 zeigt das distale Ende der Nadelschutzhülse mit dem hülsenartigen Abschnitt 14b und dem Flansch 14c. Deutlich zu sehen ist die Form des Flansches mit der konkav gewölbten Auflagefläche, deren äusserer, ovaler Rand gegenüber dem inneren Rand der Auflagefläche beim Übergang zum achsenparallelen Abschnitt nach distal vorsteht.

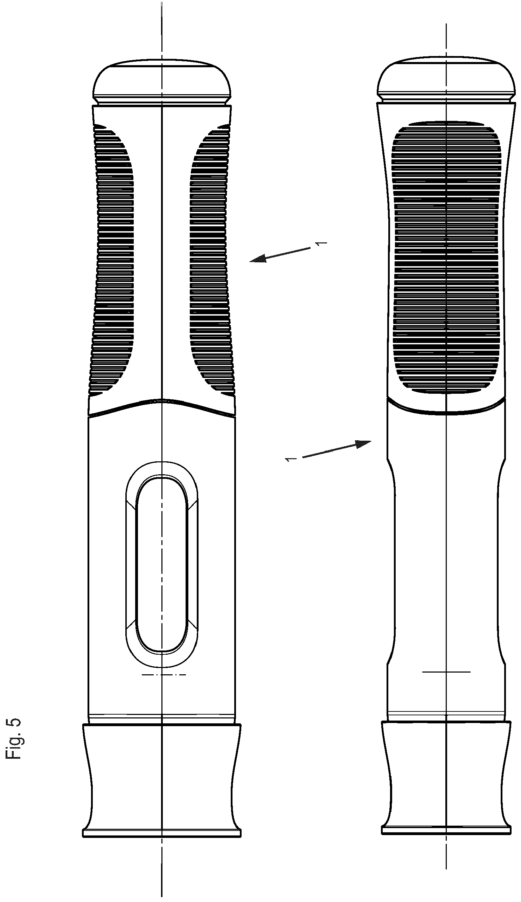

Fig. 5 Zeigt zwei um 90° gegeneinander gedrehte Ansichten einer zweiten Ausführungsform eines Autoinjektors 1 nach der Erfindung.

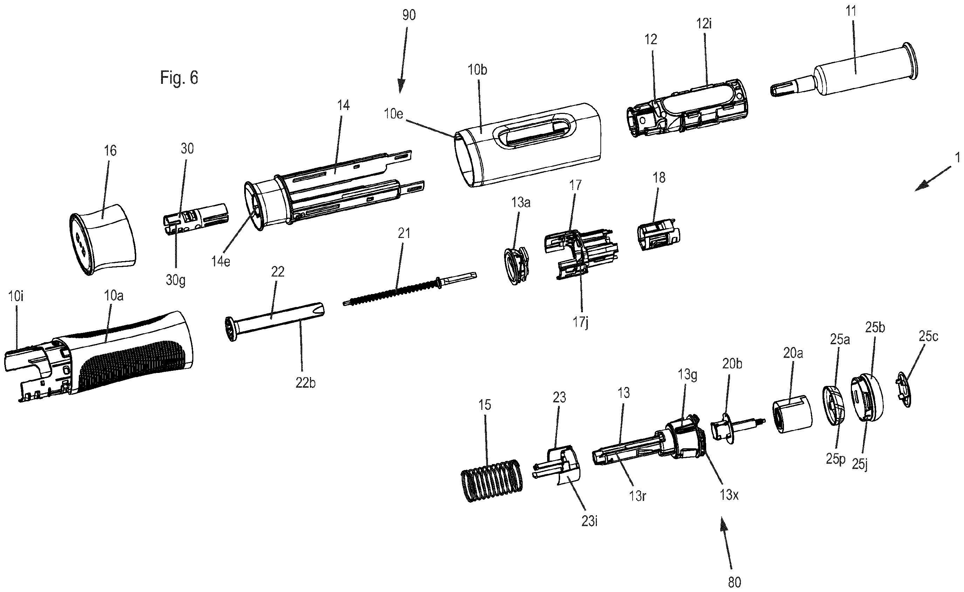

Fig. 6 ist eine Explosionsdarstellung der Komponenten einer zweiten Ausführungsform des verbesserten Autoinjektors aus Fig. 5. Der Autoinjektor 1 weist als Baugruppen eine Produktbehälteraufnahmevorrichtung 90, eine Fertigspritze 11 und eine Antriebsvorrichtung 80 auf. Dabei weist die Produktbehälteraufnahmevorrichtung 90 folgende Komponenten auf: eine Gerätekappe 16, einen Insert 30, eine Nadelschutzhülse 14, ein Spritzengehäuseteil 10b und einen Spritzenhalter 12. Die Antriebsvorrichtung 80 weist folgende Komponenten auf: ein Antriebsgehäuseteil 10a, ein Vortriebselement 22, ein Antriebselement 21, ein Spritzenadapter 13a, eine Schalthülse 17, eine Sperrhülse 18, eine Nadelschutzfeder 15, eine Kupplungshülse 23, einen Mechanikhalter 13, eine Federspule 20b, eine Spiral- oder Triebfeder 20a, ein Anzeigeelement 25a, ein Anzeigefenster 25b und eine Anzeigekappe 25c. Der Autoinjektor 1 kann lager- und/oder gebrauchsfertig aus den beiden vormontierten Baugruppen 80, 90 fertigmontiert werden, indem die Fertigspritze 11 in die Produktbehälteraufnahmevorrichtung 90 eingesetzt wird und anschliessend die Antriebsvorrichtung 80 mit der Produktbehälteraufnahmevorrichtung 90 fest verbunden, insbesondere verschnappt wird.

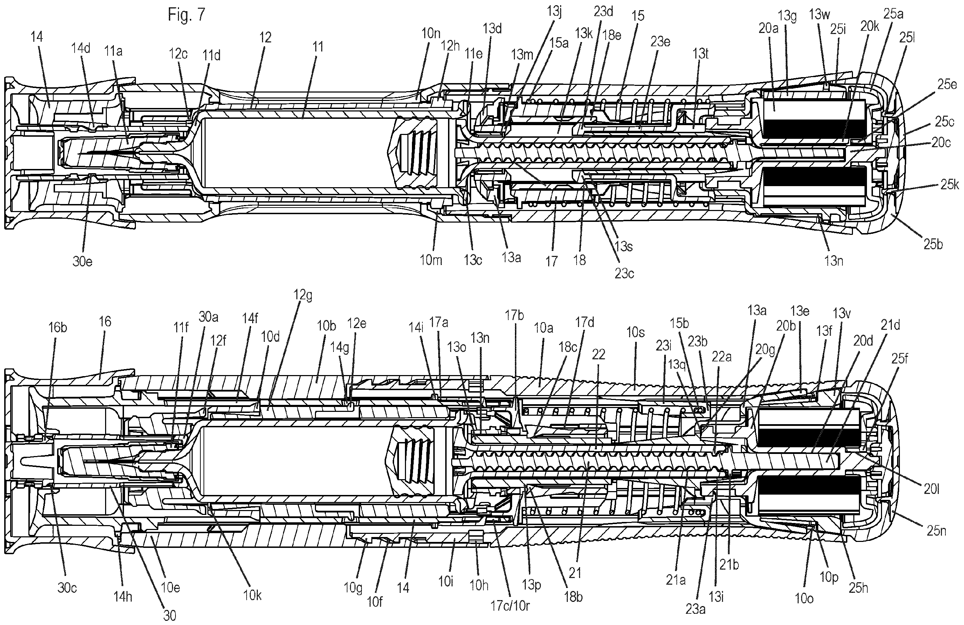

Fig. 7 (vergleiche auch Fig. 6, 11b, 12b) zeigt zwei um 90° gegeneinander gedrehte Längsschnitte des Autoinjektors aus Fig. 5 im Zustand seiner Auslieferung bzw. vor dem Gebrauch. Die Gerätekappe 16 ist durch die Haltenocken 16b, die in die korrespondierend hierzu angeordneten Halteöffnungen 30c des Insert 30 eingreifen, unlösbar zu diesem axial positioniert. Die rotative Positionierung der Gerätekappe 16 erfolgt durch deren Verdrehsicherungsrippen 16a (im Innern der Gerätekappe, nicht gezeigt), die im Eingriff mit den korrespondierend hierzu angeordneten Verdrehsicherungsnuten 30g des Insert 30, sowie den Verdrehsicherungsnuten 14e der Nadelschutzhülse 14 sind.

Zur Positionierung des Insert 30 besitzt dieser zweite Halteöffnungen 30e, in die die korrespondierend hierzu angeordneten und unter Kraftaufwand lösbaren Halteschnapper 14d der Nadelschutzhülse 14 eingreifen. Zudem sind die am proximalen Ende des Insert 30 angeordneten Haltenocken der Haltearme 30a im Eingriff mit der Schulter 11d der Nadelschutzkappe 11a der Fertigspritze 11.

Die Nadelschutzhülse 14 wird durch ihre Halteöffnungen 14f, in die die korrespondierend hierzu angeordneten Haltenocken 10d des Spritzengehäuseteils 10b eingreifen, positioniert und gegen axiales Verschieben in distale Richtung gesichert. Die Nadelschutzhülse 14 besitzt zudem weitere Halteöffnungen 14g, in die die korrespondierend hierzu angeordneten und unter Kraftaufwand lösbaren Haltenocken 12e des Spritzenhalters 12 eingreifen. Die Nadelschutzfeder 15 übt mit ihrem distalen Federende 15a eine definierte Vorspannkraft über die Schalthülse 17 auf die Nadelschutzhülse 14 aus, wodurch deren axiale Positionierung definiert wird. Die rotative Positionierung der Nadelschutzhülse 14 erfolgt durch deren Verdrehsicherungsnuten 14h, in die die korrespondierend hierzu angeordneten Verdrehsicherungsrippen 10e des Spritzengehäuseteils 10b eingreifen.

Das Spritzengehäuseteil 10b wird zusammen mit dem Antriebsgehäuseteil 10a als fixes Teil bzw. für Angaben in dieser Beschreibung als referenzbildendes Gehäuse betrachtet. Es ist durch seine Doppelschnappnute 10f, deren distale und proximale Nuten im Eingriff mit den korrespondierend hierzu angeordneten Doppelhaltenocken 10g des Antriebsgehäuseteil 10a sind, axial unlösbar zu diesem verbunden. Die rotative Positionierung des Spritzengehäuseteils 10b erfolgt durch dessen Verdrehsicherungsrippen 10h, die Kontakt zu den korrespondierend hierzu angeordneten Verdrehsicherungsflächen 10i des Antriebsgehäuseteil 10a haben.

Der Spritzenhalter 12 ist durch seine Halteschnapper 12f sowie durch seine Auflageflächen 12g, die Kontakt zu den korrespondierend hierzu angeordneten Auflageflächen 10k, 101 des Spritzengehäuseteils 10b haben, axial positioniert. Die rotative Positionierung des Spritzenhalter 12 erfolgt durch dessen Verdrehsicherungsrippen 12h, die im Eingriff mit den korrespondierend hierzu angeordneten Verdrehsicherungsnuten 10m des Spritzengehäuseteils 10b sind. Zusätzlich wird die rotative Positionierung des Spritzenhalter 12 durch seine Verdrehsicherungsrippen 12i, welche Kontakt zu dem korrespondierend hierzu ausgeführten Sichtfensterrand 10n des Spritzengehäuseteils 10b haben, gewährleistet.

Die Fertigspritze 11 ist in distale Richtung durch ihre Auflagefläche 11d, die Kontakt zu den korrespondierend hierzu angeordneten Auflagekanten 12c des Spritzenhalters 12 und in proximale Richtung durch ihre Auflagefläche 11e, die Kontakt zu der korrespondierend hierzu angeordneten Auflagefläche 13c des Spritzenadapters 13a hat, axial positioniert, wobei die flexiblen Elemente 13d des Spritzenadapters 13a, an denen die Auflagefläche 13c angebunden ist, eine definierte Vorspannung auf die Fertigspritze 11 ausüben. Rotativ ist die Fertigspritze 11 frei.

Das Spritzengehäuseteil 10b wird zusammen mit dem Antriebsgehäuseteil 10a als fixes Teil referenzbildendes Gehäuse betrachtet.

Der Mechanikhalter 13 ist durch seine Halteschnapper 13e und die Auflageflächen 13f, welche Kontakt zu den korrespondierend hierzu ausgeführten Haltenocken 10o und den Auflageflächen 10p des Antriebsgehäuseteil 10a haben, axial positioniert. Durch die Verdrehsicherungsrippen 13g, welche im Eingriff mit den korrespondierend hierzu angeordneten Verdrehsicherungsnuten 10q (nicht gezeigt) des Antriebsgehäuseteil 10a sind, wird die rotative Positionierung gewährleistet.

Die axiale Position des Vortriebselements 22 wird durch deren innenliegende Gewindepartie 22a, welche im Eingriff mit der korrespondierend hierzu ausgeführten aussenliegenden Gewindepartie 21a des Antriebselements 21 ist, definiert. Die rotative Positionierung des Vortriebselements 22 erfolgt durch deren axial sich erstreckende Verdrehsicherungsrippe 22b, welche in den korrespondierend hierzu angeordneten Verdrehsicherungsnute 13h (nicht gezeigt) des Mechanikhalters 13 geführt ist.

Das Antriebselements 21 wird durch seine umlaufende Schulter 21b, an deren distalen Ende die korrespondierend hierzu angeordneten Halteschnapper 13i des Mechanikhalter 13 eingreifen und durch ihre proximale Auflagefläche 21c, welche Kontakt zu der korrespondierend hierzu ausgeführten Auflagefläche 20c der Federspule 20b hat, axial positioniert. Die rotative Positionierung der Antriebselements 21 erfolgt durch dessen Verdrehsicherungsflächen 21d, die Kontakt zu den korrespondierend hierzu ausgeführten Verdrehsicherungsflächen 20d der Federspule 20b haben.

Der Spritzenadapter 13a wird durch seine Halteschnapper 13j, welche Kontakt zu den korrespondierend hierzu angeordneten Halteöffnungen 13k sowie durch seine Auflageflächen 13l, welche Kontakt zu den korrespondierend hierzu ausgeführten Auflageflächen 13m des Mechanikhalter 13 haben, axial positioniert. Die rotative Positionierung des Spritzenadapter 13a erfolgt durch seine Verdrehsicherungsnuten 13n, welche im Eingriff mit den korrespondierend hierzu angeordneten Verdrehsicherungsrippen 13o des Mechanikhalter 13 sind.

Die Schalthülse 17 liegt an ihrem distalen Ende mit ihren Auflageflächen 17a, welche Kontakt zu den korrespondierend hierzu ausgeführten Kontaktflächen 14i der Nadelschutzhülse 14 haben, auf und wird durch das distale Federende 15a der Nadelschutzfeder 15, welches auf der korrespondierend hierzu ausgeführten Federauflage 17b der Schalthülse 17 aufliegt, durch die definierte Federkraft der Nadelschutzfeder 15 vorgespannt. Die rotative Positionierung der Schalthülse 17 erfolgt durch deren Führungsflächen 17c, welche im Eingriff mit den korrespondierend hierzu ausgeführten Führungsrippen 10r des Antriebsgehäuseteils 10a sind.

Die Sperrhülse 18 ist innerhalb der Schalthülse 17 mit einem definierten Spiel axial positioniert. Die Sperrhülse 18 wird in distale Richtung durch ihre nach innen gerichteten Haltenocken 18b, durch Kontakt zu den korrespondierend hierzu angeordneten Auflageflächen 13p des Mechanikhalter 13, begrenzt. In proximale Richtung wird die Sperrhülse 18 durch die nach aussen gerichteten Haltenocken 18c, durch Kontakt zu den korrespondierend hierzu ausgeführten Halteöffnungen 17d der Schalthülse 17, begrenzt. Die rotative Positionierung der Sperrhülse 18 erfolgt durch deren nach innen gerichteten Führungsflächen 18d (nicht gezeigt), welche im Eingriff mit den korrespondierend hierzu ausgeführten Führungsflächen 13r des Mechanikhalter 13 sind sowie über einen Formschluss mit der Schalthülse 17.

Die Nadelschutzfeder 15 wird durch ihr distales Federende 15a, welches auf der korrespondierend hierzu ausgeführten Federauflage 17b der Schalthülse 17 und ihr proximales Federende 15b, welches auf der korrespondierend hierzu ausgeführten Federauflage 23b der Kupplungshülse 23 aufliegt, positioniert, wobei die Nadelschutzfeder 15 durch ihre Federkraft eine definierte Vorspannung zwischen der Schalthülse 17 und der Kupplungshülse 23 ausübt. Zusätzlich wird die Nadelschutzfeder 15 mit ihrer äusseren Mantelfläche durch die innenliegenden Führungsrippen 10s des Antriebsgehäuseteils 10a geführt und durch umlaufende Schürzen 23i seitlich gestützt.

Die Kupplungshülse 23 liegt mit ihren nach innen gerichteten Haltenocken 23c, welche Kontakt zu den korrespondierend hierzu angeordneten Halteflächen 13s des Mechanikhalter 13 haben an, wobei die nach aussen gerichteten Kontaktflächen 23d, welche Kontakt mit den korrespondierend hierzu ausgeführten Kontaktflächen 18e der Sperrhülse 18 haben, verhindern, dass sich die Haltearme 23e der Kupplungshülse 23 nach aussen auslenken können. Durch die definierte Vorspannung der Federkraft der Nadelschutzfeder 15, welche mit ihrem proximalen Federende 15b an der entsprechend ausgeführter Federauflage 23b anliegt, wird die axiale Position der Kupplungshülse 23 definiert. Das an der Federspule 20b anliegende Drehmoment der Spiralfeder 20a wird durch die schräggestellten Kontaktflächen 20f der Kupplungsnocken 20e, welche in Kontakt mit den korrespondierend hierzu angeordneten Kontaktflächen 23f der Kupplungsnocken 23a der Kupplungshülse 23 sind, gesperrt, wobei der Neigungswinkel der Kontaktflächen 20f , 23f so definiert wurde, dass das anliegende Drehmoment zu einem Teil als Axialkraft auf die Kupplungshülse 23 wirkt, wodurch die Kupplungshülse 23 in proximale Richtung vorgespannt wird. Die rotative Positionierung der Kupplungshülse 23 erfolgt durch die Haltearme 23e, welche im Eingriff mit den korrespondierend hierzu ausgeführten Führungsöffnungen 13t des Mechanikhalter 13 sind sowie durch den Kontakt der Kupplungsnocken 20e zu den Kupplungsnocken 23a.

Die Federspule 20b liegt mit ihrer am distalen Ende angebrachten Auflagefläche 20g an der korrespondierend hierzu ausgeführten Auflagefläche 13q des Mechanikhalter 13 an. Das an der Federspule 20b anliegende Drehmoment der Spiralfeder 20a wird durch die schräggestellten Kontaktflächen 20f der Kupplungsnocken 20e, welche in Kontakt mit den korrespondierend hierzu angeordneten Kontaktflächen 23f der Kupplungsnocken 23a der Kupplungshülse 23 sind, gesperrt, wobei der Neigungswinkel der Kontaktflächen 20f , 23f so definiert wurde, dass das anliegende Drehmoment zu einem Teil als Axialkraft auf die Federspule 20b wirkt, wodurch die Federspule 20b mit einer geringen Vorspannkraft an den Mechanikhalter 13 gedrückt und axial fixiert wird.

Die Spiralfeder 20a ist mit ihrem äusseren Federende 20h im Eingriff mit der korrespondierend hierzu ausgeführten Federaufnahme 13u des Mechanikhalter 13 und mit ihrem inneren Federende 20i im Eingriff mit der korrespondierend hierzu ausgelegten Federaufnahme 20j der Federspule 20b. Die Spiralfeder 20a ist axial zwischen Federspule 20b und Anzeigeelement 25a positioniert. Das äussere Federende 20h ist einstückig aus dem Endabschitt des Federbandes geformt. Die äusserste Windung des Federbandes ist (beispielsweise durch Punktschweissen oder Stanzbiegen oder durch eine mechanische Verankerung) zu einem hülsenförmigen Ring verbunden, wodurch ein Federgehäuse integral aus dem Federband geformt wird.

Das Anzeigeelement 25a ist durch seine Auflagefläche 25e, welche Kontakt zu der korrespondierend hierzu ausgeführten Kontaktfläche 20k sowie durch seine Haltenocken 25f, welche im Eingriff mit den korrespondierend hierzu ausgelegten Haltenocken 20l der Federspule 20b sind, axial zu diesem positioniert. Die rotative Positionierung des Anzeigeelements 25a erfolgt durch die Verdrehsicherungsrippen 20m der Federspule 20b, welche im Eingriff mit den korrespondierend hierzu ausgeführten Verdrehsicherungsnuten 25g des Anzeigeelements 25a sind.

Das Anzeigefenster 25b ist durch seine Halteöffnungen 25h, welche im Eingriff mit den korrespondierend hierzu ausgelegten Haltenocken 13v, sowie durch seine Auflagefläche 25i, welche Kontakt zu der korrespondierend hierzu ausgeführten Kontaktfläche 13w des Mechanikhalters 13 hat, axial zu diesem unlösbar positioniert. Die rotative Positionierung des Anzeigefenster 25b erfolgt durch dessen Verdrehsicherungsnuten 25j, welche im Eingriff mit den korrespondierend hierzu ausgelegten Verdrehsicherungsrippen 13x des Mechanikhalters 13 sind.

Die Anzeigekappe 25c ist durch ihre Halteschnapper 25k, welche im Eingriff mit den korrespondierend hierzu ausgeführten Halteöffnungen 25l sind sowie durch seine Auflagefläche 25m, welche in Kontakt mit der korrespondierend hierzu ausgelegten Kontaktfläche 25n ist, axial unlösbar und rotativ fix zu diesem positioniert.

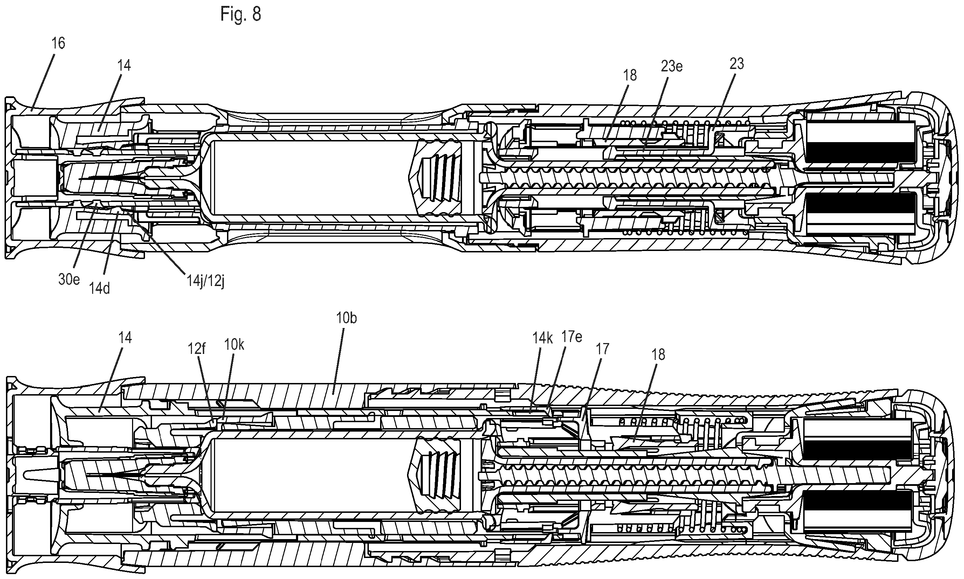

Fig. 8 zeigt zwei um 90° gegeneinander gedrehte Längsschnitte des Autoinjektors aus Fig. 5 im Zustand der Auslösesperre, welche beispielsweise durch eine starke Beschleunigung des Autoinjektors aktivierbar ist.

Wenn die Nadelschutzhülse 14 in proximale Richtung bewegt wird, werden die Halteschnapper 14d über die Halteöffnungen 30e am Insert 30 ausgelenkt. Die Sperrfläche 14j stösst an die Sperrfläche 12j des Spritzenhalters 12, welcher in proximaler Richtung durch Kontakt der Halteschnapper 12f an den Auflageflächen 10k des Spritzengehäuseteils 10b begrenzt wird. Durch die Sperrschnapper 17e, welche in die Halteöffnungen 14k eingreifen, wird die proximale Bewegung der Schalthülse 17 und somit auch der Sperrhülse 18 soweit begrenzt, dass die Haltearme 23e der Kupplungshülse 23 immer gegen ein Auslenken gesperrt sind. Somit ist gewährleistet, dass die Injektion nicht ausgelöst werden kann, solange die Gerätekappe 16 nicht entfernt wurde.

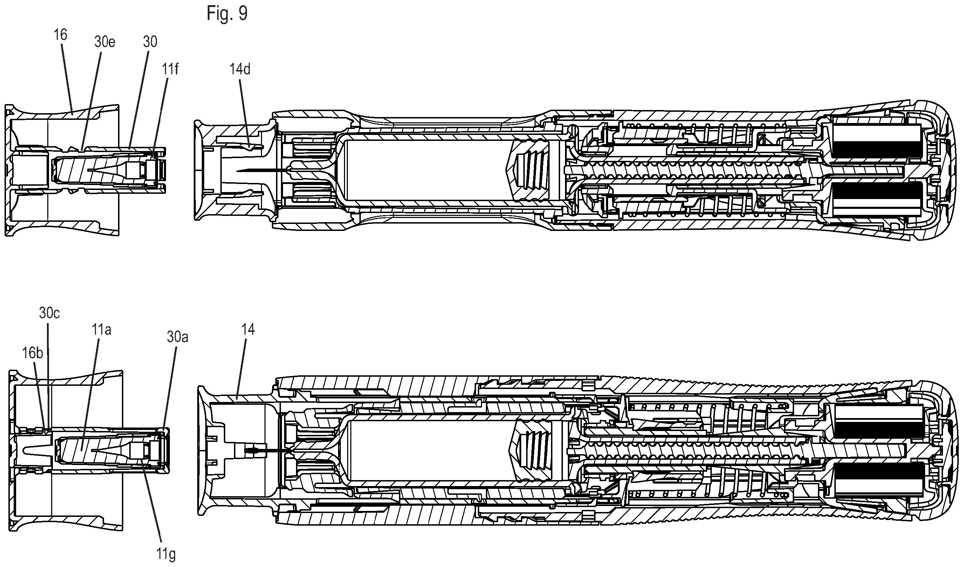

Fig. 9 zeigt zwei um 90° gegeneinander gedrehte Längsschnitte des Autoinjektors aus Fig.5 im Zustand Gerätekappe inkl. Nadelschutzkappe abgezogen.

Die Gerätekappe 16 wird im Benutzungsfall in distale Richtung vom Autoinjektor abgezogen. Der Insert 30, welcher durch seine Halteöffnungen 30c und den Haltenocken 16b der Gerätekappe 16 unlösbar zu diesem verbunden ist, wird zusammen mit der Gerätekappe 16 abgezogen. Dabei wird die unter Kraftaufwand lösbare Verbindung zwischen den Halteschnappern 14d der Nadelschutzhülse 14 und den entsprechend ausgeführten Halteöffnungen 30e des Insert 30 gelöst.

Die Nadelschutzkappe 11a, welche durch ihre Schulter 11f und die Haltearme mit Haltenocken 30a des Insert 30 unlösbar zu diesem verbunden ist, wird zusammen mit dem Gerätekappe 16 und dem Insert 30 abgezogen. Dabei wird der unter Kraftaufwand lösbare Klemmbereich 11g zwischen der Nadelschutzkappe 11a, und der Fertigspritze 11 gelöst.

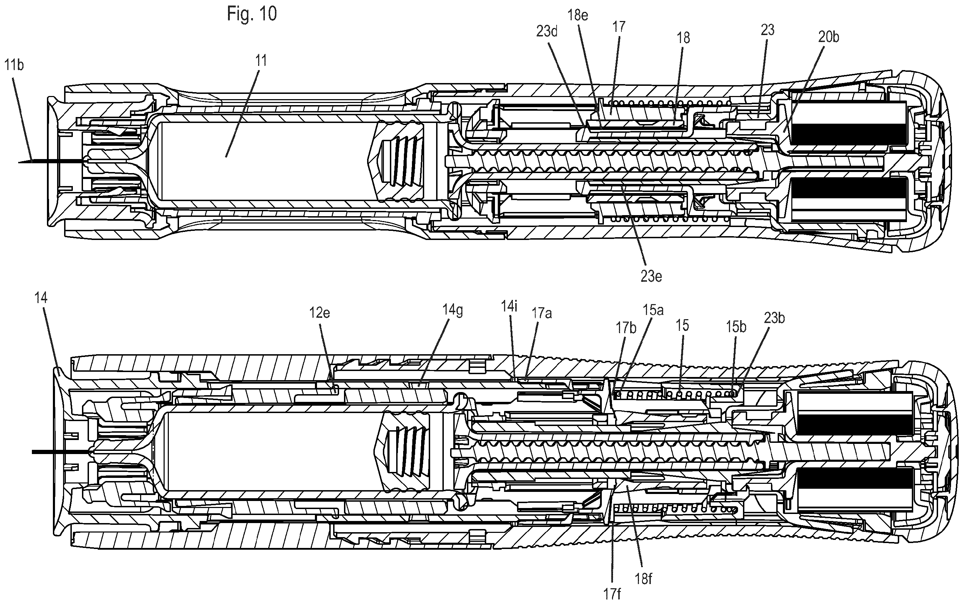

Fig. 10 zwei um 90° gegeneinander gedrehte Längsschnitte des Autoinjektors aus Fig. 5 im Zustand Eingestochen wobei das Drehmoment an der Federspule 20b noch gesperrt ist.

Um die Injektionsnadel 11b der Fertigspritze 11 in die Injektionsstelle einzustechen, wird der Autoinjektor im Benutzungsfall mit seinem distalen Ende auf die Injektionsstelle gedrückt. Während dem Nadeleinstichvorgang wird die Nadelschutzhülse 14 in proximale Richtung verschoben, wodurch die zuvor durch die Nadelschutzhülse 14 verdeckte Nadel in die Haut eingestochen wird. Zu Beginn des Nadeleinstichvorgangs muss ein definierter Auslösewiderstand überwunden werden. Hierfür löst sich die unter Kraftaufwand lösbare Verbindung zwischen den Haltenocken 12e des Spritzenhalters 12 und den korrespondierend hierzu ausgeführten Halteöffnungen 14g der Nadelschutzhülse 14. Die Schalthülse 17, die mit ihren Auflageflächen 17a in Kontakt mit den korrespondierend hierzu ausgeführten Kontaktflächen 14i der Nadelschutzhülse 14 ist, wird zusammen mit der Nadelschutzhülse 14 in proximale Richtung verschoben. Dabei wird die Nadelschutzfeder 15, die mit ihrem distalen Federende 15a auf der korrespondierend hierzu ausgeführten Federauflage 17b der Schalthülse 17 und mit ihrem proximalen Federende 15b auf der korrespondierend hierzu ausgelegten Federauflage 23b der Kupplungshülse 23 aufliegt, während der Verschiebung der Nadelschutzhülse 14 und der Schalthülse 17 vorgespannt. Die Sperrhülse 18, die mit ihren Sperrnocken 18f in Kontakt mit den korrespondierend hierzu ausgeführten Halteflächen 17f der Schalthülse 17 ist, wird zusammen mit der Nadelschutzhülse 14 und der Schalthülse 17 in proximale Richtung verschoben. Durch die Verschiebung der Sperrhülse 18 gegenüber der Kupplungshülse 23 werden die Kontaktflächen 18e der Sperrhülse 18 von den Kontaktflächen 23d der Haltearme 23e der Kupplungshülse 23 entfernt, wodurch die Haltearme 23e der Kupplungshülse 23 nicht mehr gegen Auslenken gesichert sind und somit nach aussen auslenken können.

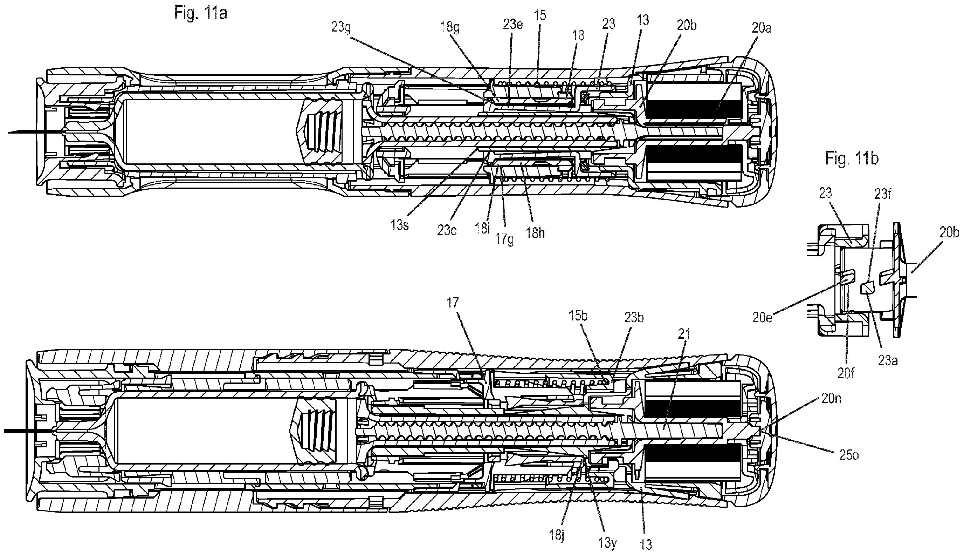

Fig. 11a zeigt zwei um 90° gegeneinander gedrehte Längsschnitte des Autoinjektors aus Fig. 5 im Zustand Freigabe Antrieb und Fig. 11b eine Ansicht im Teilschnitt der Kupplungshülse und der Federspule des Autoinjektors aus Fig. 5 im Zustand Freigabe Antrieb, unmittelbar nachdem die Federenergie freigegeben ist.

Durch die Federkraft der Nadelschutzfeder 15, welche mit ihrem proximalen Federende 15b Kontakt zur korrespondierend hierzu ausgeführten Federauflage 23b der Kupplungshülse 23 hat, wird die Kupplungshülse 23 gegenüber dem Mechanikhalters 13 und der Federspule 20b in proximale Richtung verschoben. Die durch die Sperrhülse 18 zuvor freigegebenen Haltearme 23e der Kupplungshülse 23 lenken dabei durch ihre nach innen gerichteten, schräggestellten Haltenocken 23c, welche zuvor Kontakt zu den korrespondierend hierzu ausgeführten Halteflächen 13s des Mechanikhalters 13 hatten, nach aussen aus, wodurch nun die nach aussen gerichteten Halteflächen 23g Kontakt zu den korrespondierend hierzu ausgelegten Halteflächen 18g der Haltearme 18h der Sperrhülse 18 bekommen. Der Kontakt zwischen den Kontaktflächen 18i und den korrespondierend hierzu ausgeführten Kontaktflächen 17g der Schalthülse 17 verhindert, dass die Haltearme 18h der Sperrhülse 18 nach aussen Aufspreizen können, wodurch die Kupplungshülse 23 gegenüber dem Mechanikhalters 13 und der Federspule 20b durch die Sperrhülse 18 in Position gehalten wird. Durch den Kontakt zwischen den nach aussen gerichteten Halteflächen 23g der Kupplungshülse 23 und den korrespondierend hierzu angeordneten Halteflächen 18g der Haltearme 18h der Sperrhülse 18, wird diese durch die Kupplungshülse 23 in proximale Richtung gegenüber dem Mechanikhalters 13 mitgezogen, bis sie mit ihren Auflageflächen 18j an den korrespondierend hierzu ausgeführten Auflageflächen 13y des Mechanikhalters 13 ansteht.

Durch die Verschiebung der Kupplungshülse 23 gegenüber der Federspule 20b in proximale Richtung hat sich die Kupplung zwischen den Kontaktflächen 23f der Kupplungsnocken 23a der Kupplungshülse 23 und den korrespondierend hierzu ausgeführten Kontaktflächen 20f der distal angeordneten Kupplungsnocken 20e der Federspule 20b gelöst, wodurch nun die gespeicherte Federenergie der Spiralfeder 20a freigegeben wird - Fig. 11b. Da die Federspule 20b nun nicht mehr durch die Kupplung zur Kupplungshülse 23 rotativ gesperrt und somit axial nicht mehr gegenüber dem Mechanikhalters 13 vorgespannt wird, verschiebt sich die Federspule 20b gegenüber dem Mechanikhalters 13 und dem Anzeigefenster 25b in proximale Richtung durch die Gegenkraft des Antriebselements 21, bis diese mit ihrer Auflagefläche 20n an der korrespondierend hierzu ausgeführten Auflagefläche 25o des Anzeigefensters 25b ansteht.

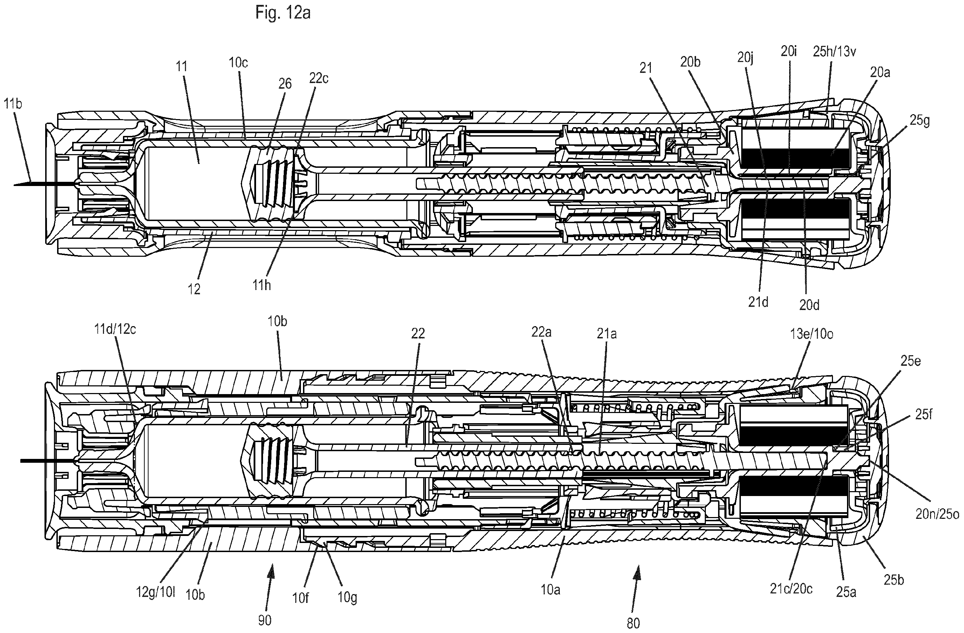

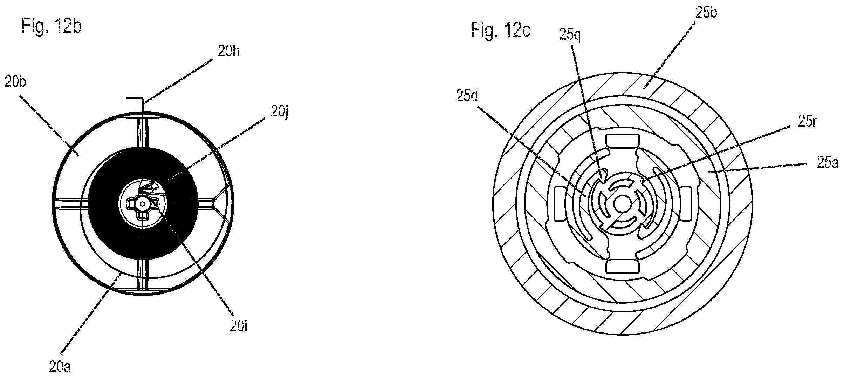

Fig. 12a zeigt zwei um 90° gegeneinander gedrehte Längsschnitte des Autoinjektors aus Fig. 5 im Zustand Ausschüttung, Fig.12b eine Ansicht proximal der Federspule 20b mit der Spiralfeder (auch Triebfeder genannt) 20a des Autoinjektors 1 aus Fig. 5 und Fig.12c einen Querschnitt aus distaler Sicht des Anzeigeelements 25a mit Anzeigefenster 25b des Autoinjektors 1 aus Fig. 5. Die in der Spiralfeder 20a gespeicherte Federenergie wird über ihr inneres Federende 20i, welches im Eingriff mit der korrespondierend hierzu ausgeführten Federaufnahme 20j der Federspule 20b ist, an diese weitergegeben, wodurch sich die Federspule 20b dreht. Die Federspule 20b gibt ihre Drehbewegung über ihre Verdrehsicherungsflächen 20d, welche Kontakt zu den korrespondierend hierzu ausgelegten Verdrehsicherungsflächen 21d des Antriebselements 21 haben, an dieses ab - Fig. 12b. Das Antriebselement 21 wiederum treibt über seine nach aussenliegende Gewindepartie 21a, welche Kontakt mit der korrespondierend hierzu ausgeführten, innenliegenden Gewindepartie 22a des Vortriebselements 22 hat, das Vortriebselement 22 in distale Richtung an, wobei ein Verdrehen der Vortriebselements 22 durch dessen äussere Verdrehsicherungsrippen 22b, welche im Eingriff mit den korrespondierend hierzu ausgelegten inneren Verdrehsicherungsnuten 13h (nicht gezeigt) des Mechanikhalters sind, verhindert wird.

Der Stopfen 26 in der Fertigspritze 11, welcher mit seiner Kontaktfläche 11h Kontakt zu der korrespondierend hierzu ausgeführten Auflagefläche 22c des Vortriebselements 22 hat, wird zusammen mit dem Vortriebselement 22 in distale Richtung verschoben, wodurch das in der Fertigspritze 11, vorhandene Medikament durch deren Injektionsnadel 11b ausgeschüttet wird.

Während der laufenden Injektion ist der Injektionsfortschritt für einen Benutzer durch das Sichtfenster 10c des Spritzengehäuseteils 10b durch Sicht auf den Stopfen 26 erkennbar. Da die Injektionsgeschwindigkeit sehr langsam und dadurch visuell nur schwer wahrnehmbar sein kann (beispielsweise 60 Sekunden Injektionsdauer für eine vollständig gefüllte Fertigspritze), ist eine zusätzliche Injektionsbewegungsanzeige am proximalen Ende des Autoinj ektors integriert. Das Anzeigeelement 25a, mit Auflagefläche 25e an die korrespondierend hierzu ausgeführten Kontaktflächen 20k der Federspule stossend, mittels Haltenocken 25f axial verschnappt mit den Haltenocken 201 der Federspule 20b sowie eingreifend durch seine Verdrehsicherungsnuten 25g in den Verdrehsicherungsrippen 20m der Federspule 20b, dreht sich zusammen mit der Federspule 20b gegenüber dem Anzeigefenster 25b während der Injektion. Diese Drehbewegung des Anzeigeelements 25a durch ein aufgebrachtes Muster 25p für einen Benutzer durch das transparente Anzeigefenster 25b sichtbar, wodurch einem Benutzer ein zusätzliches und verbessertes visuelles Feedback zur laufenden Injektion zur Verfügung steht.

Ergänzend zu dem visuellen Feedback erhält eine Benutzerin ein kontinuierliches, akustisches Feedback zur laufenden Injektion in Form von Klickgeräuschen, die zwischen dem sich drehenden Anzeigeelement 25a und dem stationären Anzeigefenster 25b erzeugt werden. Die an den Federamen 25d des Anzeigeelement 25a angebrachten Rastnocken 25q gleiten während der Injektion über die korrespondierend hierzu ausgeführte Rasterung 25r des Anzeigefensters 25b, wobei die Federarme 25d des Anzeigeelements 25a während der Drehung gegenüber dem Anzeigefenster 25b radial vorgespannt und wieder entspannt werden, wodurch für eine Benutzerin ein kontinuierliches, akustisches Klicken wahrnehmbar wird. Falls die Klickfrequenz an eine Ausschüttgeschwindigkeit angepasst werden soll, kann die Anzahl der Rasterung 25r variiert werden. Dabei gilt es zu berücksichtigen, dass die Teilung mit den Montagepositionen der Federarme 25d übereinstimmt. Vorstellbar wären beispielsweise 2, 4 oder 8 Rasterungen je Umdrehung. - Fig. 12c zeigt eine Ausführung mit 4 Rasterungen 25r und zwei Federarmen 25d.

Die Injektionskraft drückt die beiden Unterbaugruppen Produktbehälteraufnahmevorrichtung 90 und Antriebsvorrichtung 80 auseinander, welche über die Doppelschnappnute 10f und Doppelhaltenocken 10g an Spritzengehäuseteil 10b beziehungsweise Antriebsgehäuseteil 10a zusammengehalten werden. In distale Richtung wird die Injektionskraft über die Schnittstellen 11d / 12c und 12g / 101 von der Fertigspritze 11 über den Spritzenhalter 12 auf das Spritzengehäuseteil 10b übertragen. In proximale Richtung wird die Injektionskraft über die Schnittstellen 22a / 21a, 21c / 20c, 20n / 25o, 25h / 13v und 13e / 10o von dem Vortriebselement 22 über das Antriebselement 21, die Federspule 20b, das Anzeigefenster 25b und den Mechanikhalter 13 auf das Antriebsgehäuseteil 10a übertragen.

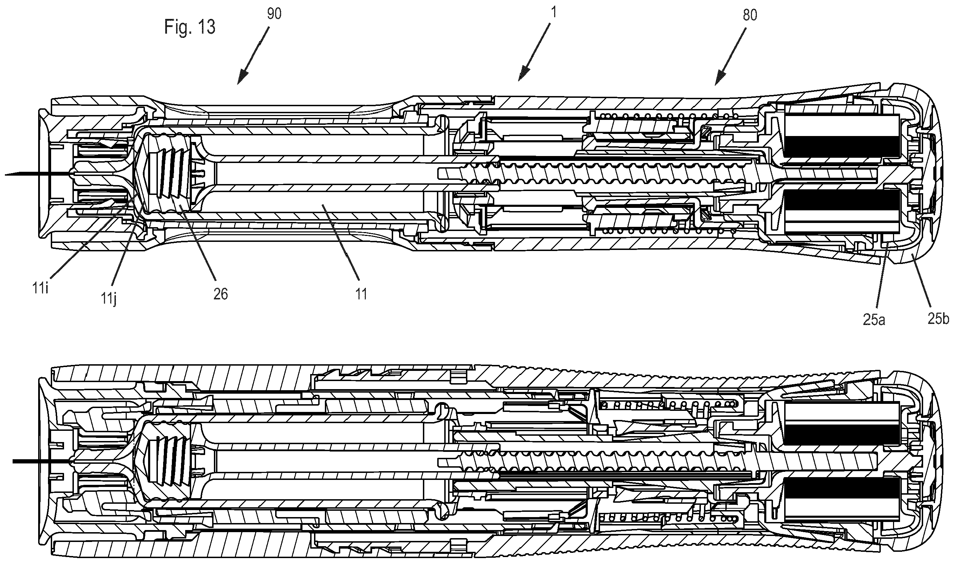

Fig. 13 zeigt zwei um 90° gegeneinander gedrehte Längsschnitte des Autoinjektors aus Fig. 5 im Zustand Ausschüttung beendet.

Die Verschiebung des Stopfen 26 in distale Richtung stoppt automatisch, sobald dieser mit seiner am distalen Ende angeordneten Kontaktfläche 11j an der korrespondierend hierzu ausgeführten Kontaktfläche 11i der Fertigspritze 11 anschlägt. Die automatische Injektion ist somit beendet. Die beendete Ausschüttung ist für einen Benutzer durch das bewegungslose Anzeigeelement 25a ersichtlich und die Abwesenheit des Klickgeräuschs wahrnehmbar, welches durch die relative Bewegung von Anzeigeelements 25a und gehäusefestem Anzeigefenster 25b bewirkt wird.

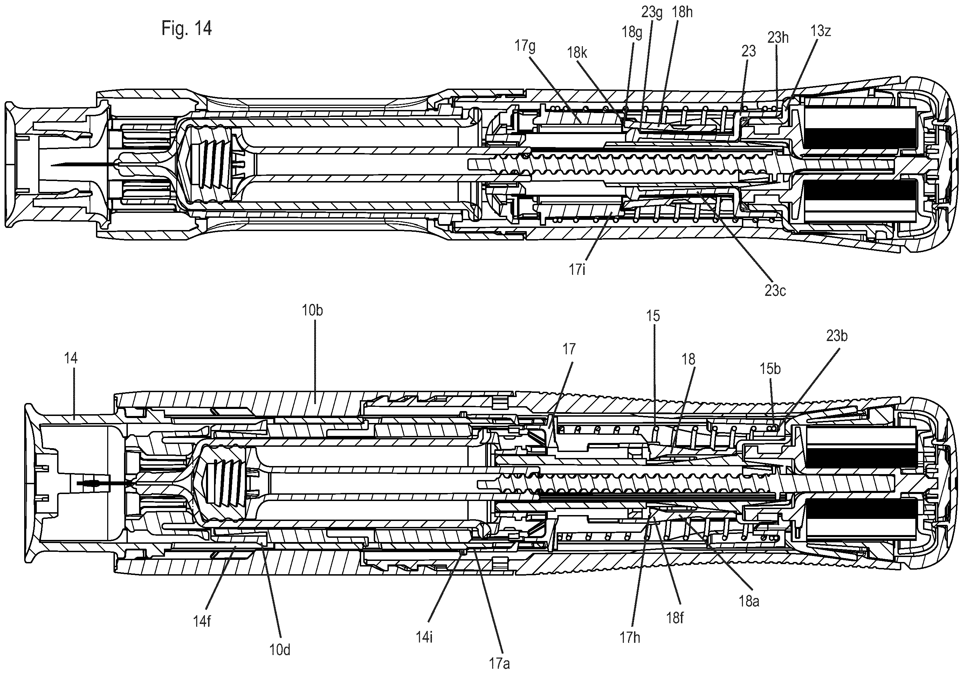

Fig. 14 zeigt zwei um 90° gegeneinander gedrehte Längsschnitte des Autoinjektors aus Fig. 5 im Zustand Nadelschutzverriegelung aktiviert.

Nach erfolgter Injektion bzw. beendeter Ausschüttung wird die Nadelschutzhülse 14 durch die Federkraft der Nadelschutzfeder 15, zusammen mit der Schalthülse 17 wieder in ihre ursprüngliche Position in distale Richtung verschoben und durch die Sperrhülse 18 gegen nochmaliges Einstechen gesperrt. Die nach aussen gerichteten Sperrnocken 18f der Sperrarme 18a, sowie die Sperrflächen 18k der nach aussen ausgelenkten Haltearme 18h der Sperrhülse 18 kommen in Kontakt mit den korrespondierend hierzu ausgeführten Sperrflächen 17h sowie den Sperrflächen 17i der Schalthülse 17, wodurch ein nochmaliges Verschieben der Schalthülse 17 und somit der Nadelschutzhülse 14 in proximale Richtung verhindert wird indem die Auflageflächen 17a in formschlüssigen Kontakt mit den korrespondierend hierzu ausgeführten Kontaktflächen 14i sind.

Während dem Entfernen des Autoinjektors 1 von der Injektionsstelle wird durch die Federkraft der Nadelschutzfeder 15, welche mit ihrem distalen Federende 15a in Kontakt mit der korrespondierend hierzu ausgeführten Federauflage 17b der Schalthülse 17 und diese wiederum durch ihre Auflageflächen 17a in Kontakt mit den korrespondierend hierzu ausgelegten Kontaktflächen 14i der Nadelschutzhülse 14 ist, die Nadelschutzhülse 14 sowie die Schalthülse 17 wieder in ihre ursprüngliche Positionen distal verschoben, bis die Halteöffnungen 14f der Nadelschutzhülse 14 wieder in Kontakt mit den Haltenocken 10d des Spritzengehäuseteil 10b kommen.

Die Schalthülse 17 wird durch die Federkraft der Nadelschutzfeder 15 zusammen mit der Nadelschutzhülse 14 in distale Richtung in ihre ursprüngliche Position verschoben. Während der Verschiebung der Schalthülse 17 gegenüber der Sperrhülse 18 werden kurzzeitig die Sperrarme 18a der Sperrhülse 18 nach innen ausgelenkt, bis sich die Schalthülse 17 komplett von den Sperrnocken 18f der Sperrarme 18a entfernt hat, wodurch nun die Sperrnocken 18f der Sperrarme 18a hinter dem proximalen Ende der Schalthülse 17 zu liegen kommen.

Die Verschiebung der Schalthülse 17 gegenüber der Sperrhülse 18 bewirkt, dass deren Haltearme 18h, welche zuvor durch ihre Kontaktflächen 18i, welche Kontakt zu den korrespondierend hierzu angeordneten Kontaktflächen 17g der Schalthülse 17 hatten und somit gegen Auslenken gesichert waren, nun nach aussen auslenken können. Die durch die Halteflächen 23g der Haltearme 23e der Kupplungshülse 23, welche zuvor zu den korrespondierend hierzu ausgelegten Halteflächen 18g der Haltearme 18h der Sperrhülse 18 Kontakt hatten und somit gegen Verschieben gesicherte Kupplungshülse 23 ist nun in proximale Richtung gegenüber der Sperrhülse 18 und dem Mechanikhalter 13 verschiebbar.

Durch die Federkraft der Nadelschutzfeder 15, welche mit ihrem proximalen Federende 15b in Kontakt mit der korrespondierend hierzu ausgeführten Federauflage 23b der Kupplungshülse 23 ist, wird nun die Kupplungshülse 23 gegenüber der Sperrhülse 18 in proximale Richtung verschoben, bis sie mit ihren Auflageflächen 23h an den korrespondierend hierzu ausgelegten Auflageflächen 13z des Mechanikhalter 13 ansteht. Dabei werden durch die Halteflächen 23g der Haltearme 23e der Kupplungshülse 23 die Haltearme 18h der Sperrhülse 18 nach aussen ausgelenkt.

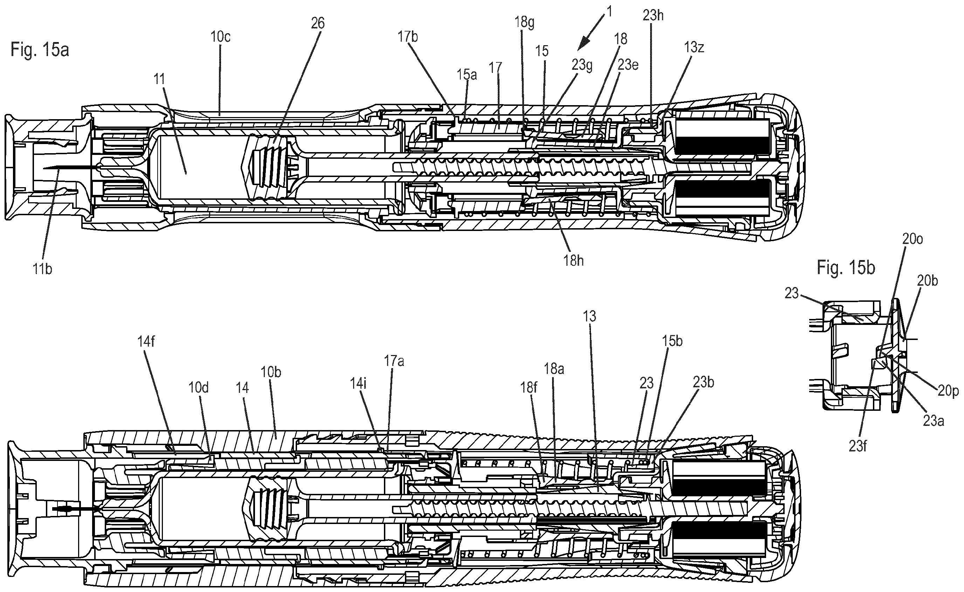

Fig. 15a zeigt zwei um 90° gegeneinander gedrehte Längsschnitte des Autoinjektors aus Fig. 5 im Zustand Ausschüttung abgebrochen und Fig.15b eine Ansicht im Teilschnitt der Kupplungshülse und der Federspule des Autoinjektors aus Fig. 5 im Zustand Ausschüttung abgebrochen.

Ein Injektionsabbruchs bzw. ein Stopp der Ausschüttung erfolgt, falls ein Benutzer den Autoinjektor 1 von der Injektionsstelle entfernt bevor die Ausschüttung abgeschlossen ist, wodurch auch die automatische Nadelschutzverriegelung aktiviert wird. Gleichzeitig wird die automatische Ausschüttung gestoppt und somit ein unerwünschtes Nachtropfen des restlichen Medikaments aus der Injektionsnadel 11b verhindert.

Während dem Entfernen des Autoinjektors von der Injektionsstelle wird durch die Federkraft der Nadelschutzfeder 15, welche mit ihrem distalen Federende 15a in Kontakt mit der korrespondierend hierzu ausgeführten Federauflage 17b der Schalthülse 17 und diese wiederum durch ihre Auflageflächen 17a in Kontakt mit den korrespondierend hierzu ausgelegten Kontaktflächen 14i der Nadelschutzhülse 14 ist, die Nadelschutzhülse 14 sowie die Schalthülse 17 wieder in ihre ursprüngliche Positionen distal verschoben, bis die Halteöffnungen 14f der Nadelschutzhülse 14 wieder in Kontakt mit den Haltenocken 10d des Spritzengehäuseteils 10b kommen.

Die Schalthülse 17 wird durch die Federkraft der Nadelschutzfeder 15 zusammen mit der Nadelschutzhülse 14 in distale Richtung in ihre ursprüngliche Position verschoben. Während der Verschiebung der Schalthülse 17 gegenüber der Sperrhülse 18 werden kurzzeitig die Sperrarme 18a der Sperrhülse 18 nach innen ausgelenkt, bis sich die Schalthülse 17 komplett von den Sperrnocken 18f der Sperrarme 18a entfernt hat, wodurch nun die Sperrnocken 18f der Sperrarme 18a hinter dem proximalen Ende der Schalthülse 17 zu liegen kommen.

Die Verschiebung der Schalthülse 17 gegenüber der Sperrhülse 18 bewirkt, dass deren Haltearme 18h, welche zuvor durch ihre Kontaktflächen 18i, welche Kontakt zu den korrespondierend hierzu angeordneten Kontaktflächen 17g der Schalthülse 17 hatten und somit gegen Auslenken gesichert waren, nun nach aussen auslenken können. Die durch die Halteflächen 23g der Haltearme 23e der Kupplungshülse 23, welche zuvor zu den korrespondierend hierzu ausgelegten Halteflächen 18g der Haltearme 18h der Sperrhülse 18 Kontakt hatten und somit gegen Verschieben gesicherte Kupplungshülse 23, kann nun in proximale Richtung gegenüber der Sperrhülse 18 und dem Mechanikhalter 13 verschieben.

Durch die Federkraft der Nadelschutzfeder 15, welche mit ihrem proximalen Federende 15b in Kontakt mit der korrespondierend hierzu ausgeführten Federauflage 23b der Kupplungshülse 23 ist, wird nun die Kupplungshülse 23 gegenüber der Sperrhülse 18 in proximale Richtung verschoben, bis sie mit ihren Auflageflächen 23h an den korrespondierend hierzu ausgelegten Auflageflächen 13z des Mechanikhalter 13 ansteht. Dabei werden durch die Halteflächen 23g der Haltearme 23e der Kupplungshülse 23 die Haltearme 18h der Sperrhülse 18 nach aussen ausgelenkt.

Die Verschiebung der Kupplungshülse 23 in proximale Richtung gegenüber der Federspule 20b bewirkt, dass deren Kontaktflächen 23f der Kupplungsnocken 23a in Eingriff mit den korrespondierend hierzu ausgeführten Kontaktflächen 20o der proximal angeordneten Kupplungsnocken 20p der Federspule 20b kommen - Fig. 15b. Hierdurch wird die Drehung der Federspule 20b blockiert und die automatische Ausschüttung stoppt.

Nach einer durch eine Benutzerin abgebrochenen Injektion ist durch das Sichtfenster 10c des Spritzengehäuseteil die axiale Position des Stopfen 26 der Fertigspritze 11 ersichtlich, wodurch eine Benutzerin abschätzen kann, wieviel Injektionsvolumen abgegeben wurde respektive wieviel Restmedikament noch übrig ist.

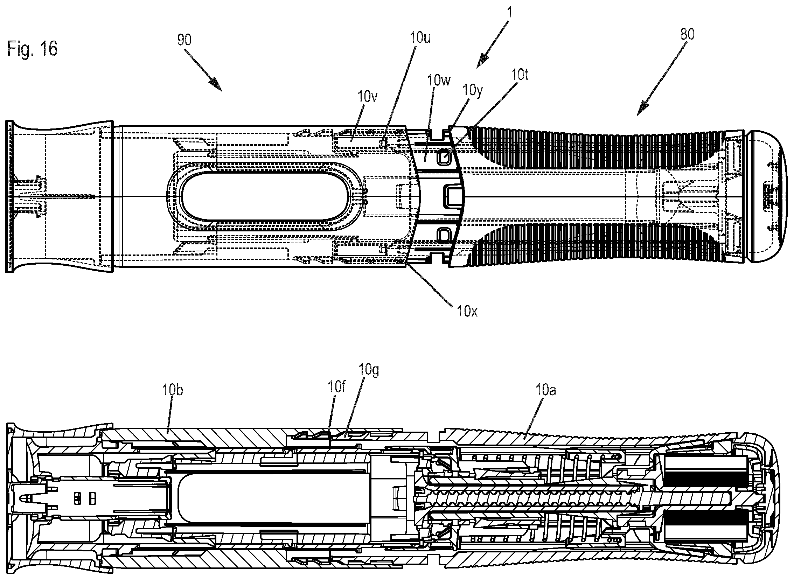

Fig. 16 zeigt eine Ansicht und einen Längsschnitt des Autoinjektors 1 aus Fig. 5 im Zustand Vorverschnappt. Der Zustand Vorverschnappt ist Ausgangszustand für die Montage einer Fertigspritze und die Endmontage des Autoinjektors 1. Im Zustand Vorverschnappt sind die beiden Unterbaugruppen Antriebsvorrichtung 80 und Produktbehälteraufnahmevorrichtung 90 trennbar miteinander verbunden und können durch die kraftschlüssige axiale und formschlüssige rotative Fixierung sicher in definierter Lage zueinander gelagert und transportiert werden. Die Unterbaugruppen Produktbehälteraufnahmevorrichtung 90 und Antriebsvorrichtung 80 sind mittels der lösbaren Schnappverbindung 10v / 10u zwischen Spritzengehäuseteil 10b und Antriebsgehäuseteil 10a vorverschnappt. Dazu greift die Rastnocke 10u am Antriebsgehäuseteil 10a hinter den proximalen Rand der Rastnute 10v im Spritzengehäuseteil 10b. Weiter stösst die distale Seite der Doppelhaltenocken 10g an den proximalen Rand der Doppelhaltenute 10f und positioniert damit die Gehäuseteile 10a, 10b axial relativ zueinander in einem definierten Abstand wie in Fig. 16 gezeigt. Eine umlaufende Stufe 10y leitet am Antriebsgehäuseteil 10a einen distalen Endabschitt 10w ein. Die Montagefenster 10t im distalen Endabschnitt 10w des Antriebsgehäuseteils 10a sind von aussen sichtbar und zugänglich. Der Endabschnitt 10w ist teilweise in das proximale Ende des Spritzengehäuseteils 10b eingeschoben, wobei sich zwischen den beiden Gehäuseteilen 10a, 10b im Bereich des Endabschnitts 10w eine umlaufende axial wirkende Spaltführung 10x ausbildet.

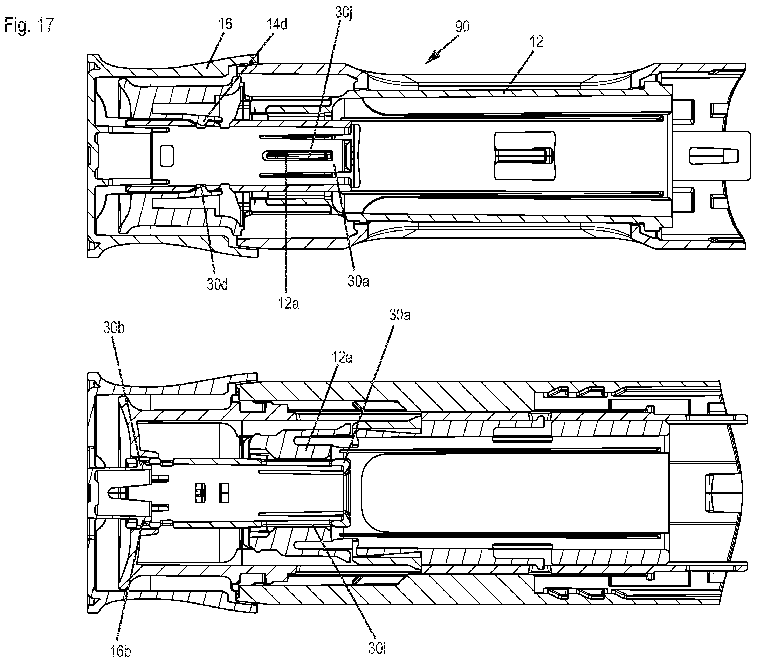

Fig. 17 zeigt zwei um 90° gegeneinander gedrehte Längsschnitte der Produktbehälteraufnahmevorrichtung 90 mit Insert 30 vormontiert. Die Antriebsvorrichtung 80 wurde in einem ersten Schritt vom vorverschnappten Autoinjektor entfernt und die Produktbehälteraufnahmevorrichtung 90 ist damit bereit, eine Fertigspritze in einem Fügevorgang durch die proximale Öffnung aufzunehmen. Der Insert 30 ist mit seinen distalen Halteöffnungen 30b im vormontierten Zustand im Eingriff mit den korrespondierend hierzu ausgeführten Haltenocken 16b der Gerätekappe 16. Zudem greifen die unter Kraftaufwand lösbaren Halteschnapper 14d der Nadelschutzhülse 14 in die korrespondierend hierzu ausgelegten Halteöffnungen 30d des Insert 30 ein, wodurch dieser in Bezug zu den andern Teilen der Produktbehälteraufnahmevorrichtung 90 positioniert wird. Die Halteschlitze 30j der Haltearme 30a sind in diesem Zustand so zu den korrespondierend hierzu angeordneten Halterippen 12a des Spritzenhalters 12 positioniert, dass die flexiblen Haltearme mit den Haltenocken 30a nach aussen auslenken können, wobei die Halterippen 12a des Spritzenhalters 12 in die Halteschlitze 30j der Haltearme 30a einfahren bzw. die Haltearme 30a in den axial laufenden Ausnehmungen neben den Halterippen 12a Platz finden.

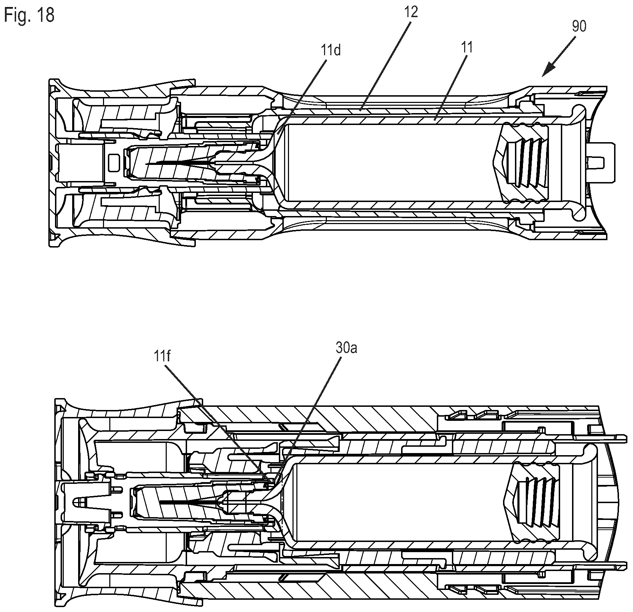

Fig. 18 zeigt zwei um 90° gegeneinander gedrehte Längsschnitte der Produktbehälteraufnahmevorrichtung 90 mit Fertigspritze 11 (PFS mit RNS) vormontiert. Die Fertigspritze 11 wird soweit axial in distale Richtung in die Produktbehälteraufnahmevorrichtung 90 gefügt, bis diese mit ihrer Auflagefläche 11d in Kontakt mit den korrespondierend hierzu ausgeführten Haltearmen mit Haltenocken 30a sowie den Auflageflächen 30h des Insert 30 kommt. Während diesem Fügevorgang der Fertigspritze 11 gleiten die Haltenocken der flexiblen Haltearme 30a des Insert 30 über die Mantelfläche der Nadelschutzkappe 11a der Fertigspritze 11, bis die Haltenocken am Haltearm 30a proximal der Schulter 11f der Nadelschutzkappe 11a elastisch einschnappen oder zu liegen kommen.

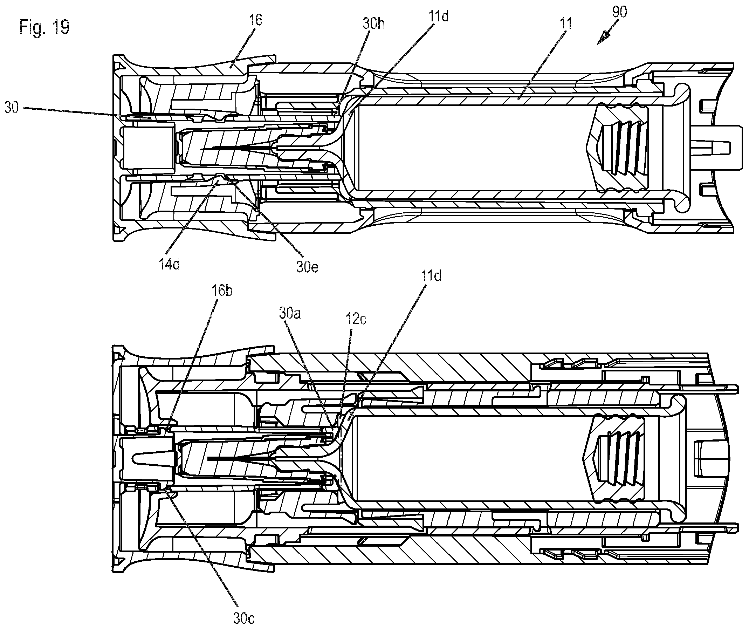

Fig. 19 zeigt zwei um 90° gegeneinander gedrehte Längsschnitte der Produktbehälteraufnahmevorrichtung 90 mit Insert 30 und Fertigspritze 11 (PFS mit RNS) endmontiert. Für diesen Endmontageschritt wird die Fertigspritze 11 weiter axial in distale Richtung in den Autoinjektor gefügt, bis deren Auflageflächen oder Schulter 11d in Kontakt mit den korrespondierend hierzu angeordneten Auflagekanten 12c des Spritzenhalters 12 kommen. Durch den Kontakt der Auflagefläche 11d der Fertigspritze 11 zu den korrespondierend hierzu ausgeführten Haltenocken 30a sowie den stirnseitigen Auflageflächen 30h des Insert 30 wird dieser gegenüber dem Gerätekappe 16 in distale Richtung mitgeschoben. Dabei wechseln die Haltenocken 16b von den distalen Halteöffnungen 30b zu den proximalen Halteöffnungen 30c. Zudem wechselt der Halteschnapper 14d der Nadelschutzhülse 14 von der Halteöffnung 30d in die korrespondierend hierzu ausgelegte proximale Halteöffnungen 30e des Insert 30. Durch diesen Endmontageschritt gelangen die Haltearme mit den Haltenocken 30a nun unter die Halterippen 12a des Spritzenhalters 12 bzw. die Halteschlitze 30j der Haltearme stellen die Halterippen 12a des Spritzenhalters 12 nicht mehr frei, wodurch verhindert wird, dass die flexiblen Haltearme mit den Haltenocken 30a des Insert 30 nach aussen auslenken können. Somit ist der Eingriff der Haltenocken 30a zu der RNS Schulter 11f der Fertigspritze 11 gesichert formschlüssig.

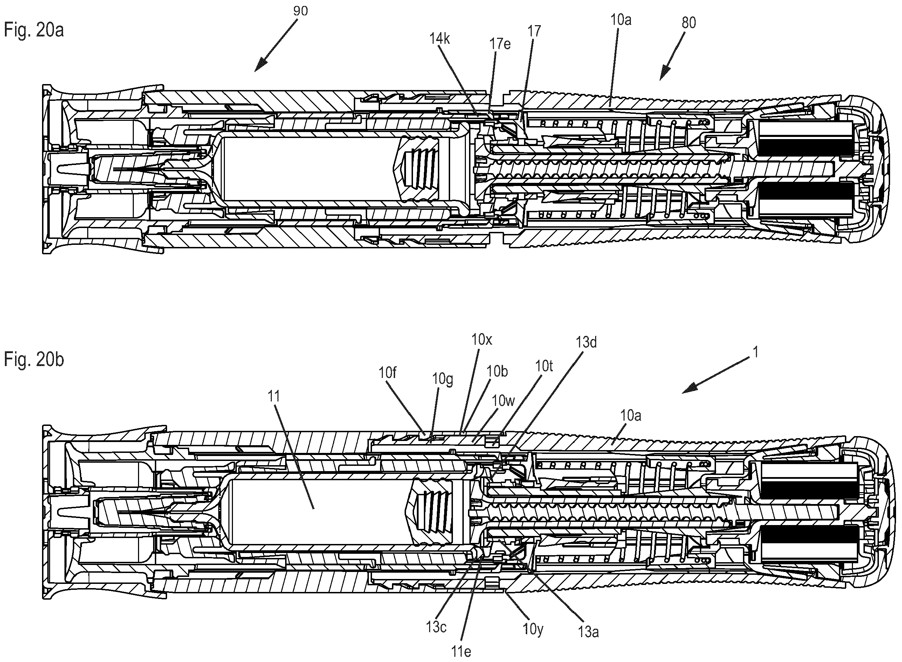

Fig. 20a zeigt einen Längsschnitt der Produktbehälteraufnahmevorrichtung 90 mit montierter Fertigspritze 11 nach einem ersten Fügeschritt der Antriebsvorrichtung 80. Während diesem ersten Fügeschritt kann ein Montagewerkzeug (nicht gezeigt) durch die Montagefenster 10t (siehe Fig. 16) seitlich eingesetzt sein und die Schalthülse 17 durch Eingriff in die Montagenute 17j (siehe Fig. 6) axial fixieren gegenüber dem Gehäuseteil 10a womit sichergestellt wird, dass der Sperrschnapper 17e an der festgehaltenen Schalthülse 17 während dem ersten Fügeschritt in die Halteöffnung 14k der Nadelschutzhülse 14 einlenken kann.

Fig. 20b zeigt einen Längsschnitt der Produktbehälteraufnahmevorrichtung 90 mit montierter Fertigspritze 11 nach einem zweiten finalen Fügeschritt der Antriebsvorrichtung 80. Vor diesem zweiten Fügeschritt wird das Montagewerkzeug (nicht gezeigt) entfernt. Die Antriebsvorrichtung 80 wird in diesem zweiten Fügeschritt axial in einer linearen Bewegung entlang der Spaltführung 10x bis auf Anschlag an der Stufe 10y in die Produktbehälteraufnahmevorrichtung 90 eingeschoben, wobei die Doppelhaltenocken 10g in ihre jeweils korrespondierenden Doppelschnappnuten 10f einschnappen und die Antriebsvorrichtung 80 formschlüssig und damit unlösbar mit der Produktbehälteraufnahmevorrichtung 90 verbinden. Durch den zweiten Fügeschritt werden die Montagefenster 10t am distalen Endabschnitt 10w des Antriebsgehäuseteils 10a durch das umlaufende hülsenförmige Ende des Spritzengehäuseteils 10b abgedeckt, wodurch die Aussenhülle des endmontierten Autoinjektors 1 nicht durch störende Öffnungen durchbrochen wird und das Innere des Autoinjektors 1 geschützt bleibt. Die Auflagefläche 13c des Spritzenadapters 13a wird durch die Auflagefläche 11e am proximalen Ende der Fertigspritze 11 axial positioniert, wobei die flexiblen Elemente 13d des Spritzenadapters 13a, an denen die Auflagefläche 13c angebunden ist, durch den zweiten Fügeschritt vorgespannt werden.

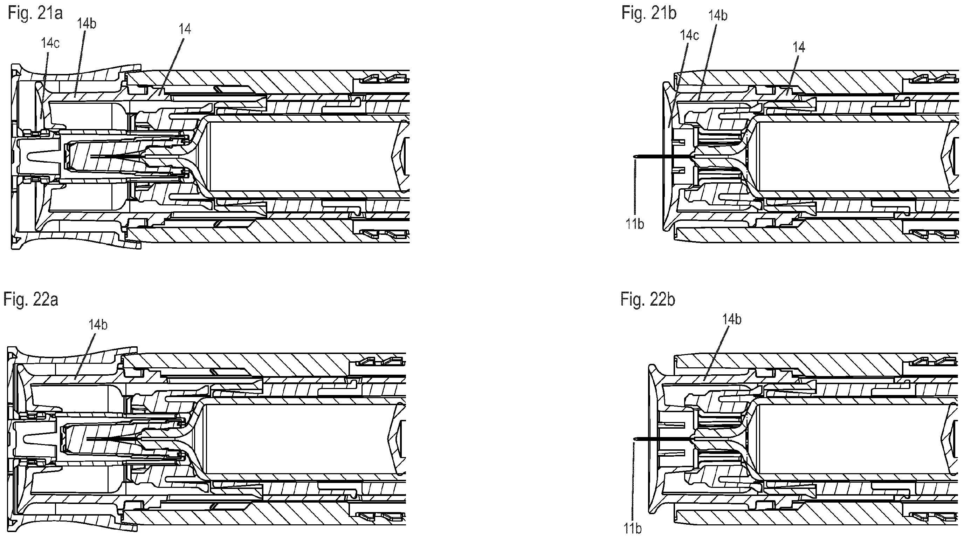

Mit Autoinjektorvarianten sollen Einstichtiefen der Nadel in die Haut im von beispielsweise etwa 3-15 mm möglich sein. Fig. 21a zeigt eine Autoinjektorvariante für eine beispielhafte Einstechtiefe von 8 mm im Zustand Auslieferung und Fig. 21b im Zustand Eingestochen. Fig. 22a zeigt eine Autoinjektorvariante für eine beispielhafte Einstechtiefe von 5 mm im Zustand Auslieferung und Fig. 22b im Zustand Eingestochen. Diese beiden und weitere Varianten unterscheiden sich nur durch unterschiedliche Nadelschutzhülsen 14 bei denen jeweils der axial sich erstreckende Abschnitt 14b kürzer oder länger ausgebildet ist wodurch der auf die Haut aufzusetzende Flansch 14c unterschiedlich positioniert wird, womit die Einstechtiefe der Nadel 11b entsprechend begrenzt wird.

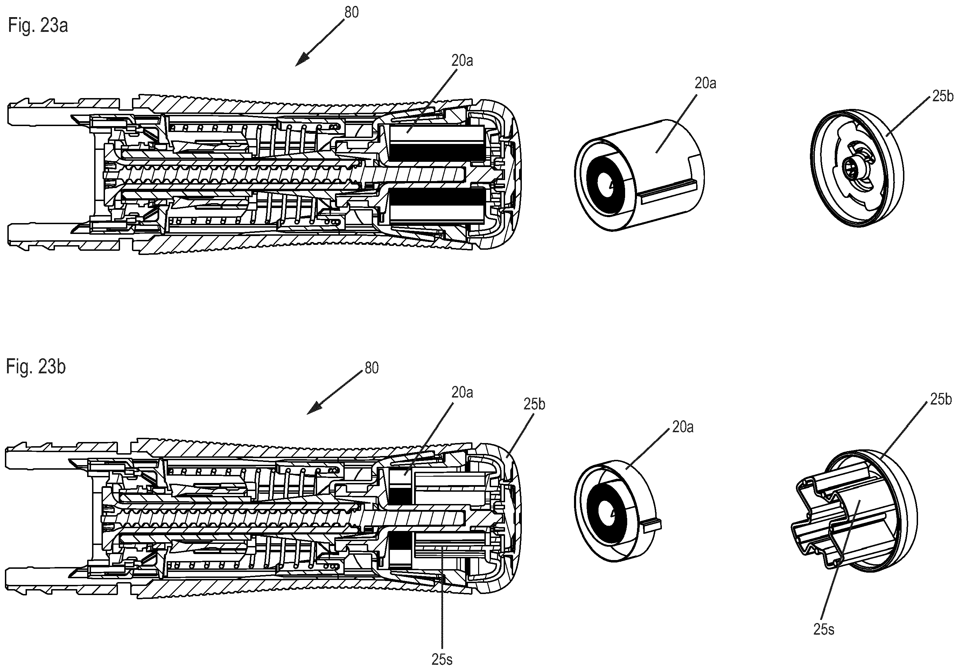

Fig. 23a und 23b zeigen je einen Längsschnitt durch das proximale Ende der Antriebsvorrichtung 80 sowie je eine Ansicht der Triebfeder 20a und des Anzeigefensters 25b. Um unterschiedliche Drehmomente bzw. Injektionskräfte zu erreichen, können verschiedene Triebfedern verbaut werden. Die Triebfedern haben dabei unterschiedliche Federbandbreiten und liefern somit unterschiedlich hohe Federdrehmomente. Komplementär zur Federbandbreite können unterschiedlich lang axial sich erstreckende Füllprofile 25s distal am Anzeigefensters 25b vorgesehen sein, damit der Einbauraum für die Feder axial begrenzt wird. Dadurch wird ein Ausbrechen der Federspirale in axialer Richtung vermieden.

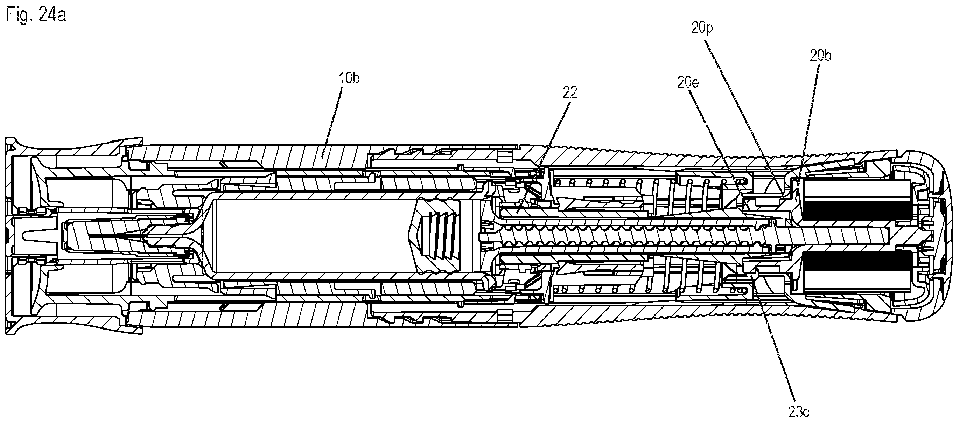

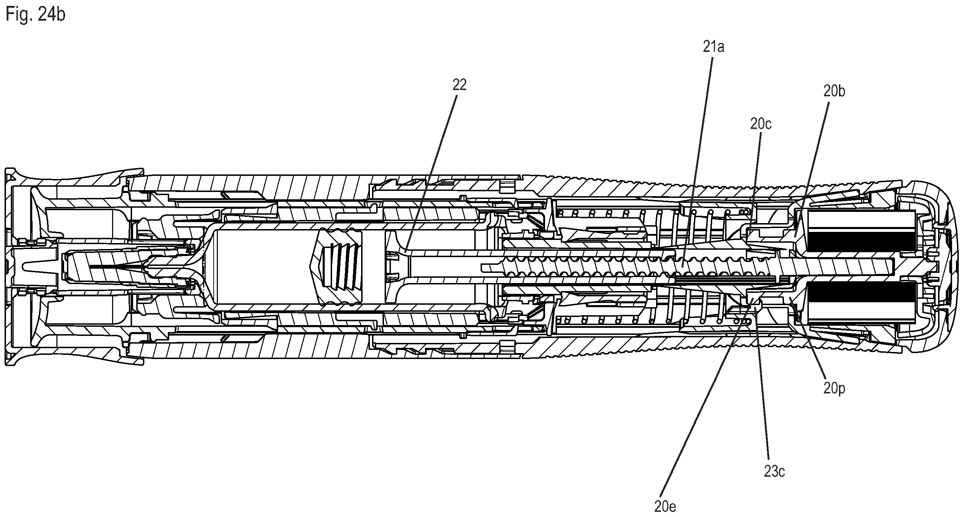

Fig. 24a und Fig. 24b zeigen je einen Längsschnitt durch Autoinjektoren mit Fertigspritzen, welche mit unterschiedlichen Volumen an Medikament vorgefüllt sind. Bei voll gefüllter Spritze - Fig. 24a, ist der Hub des Vortriebselements 22 maximal. Bei nur teilweise gefüllter Spritze - Fig. 24b, kann der Hub des mittels unterschiedlichen Montagepositionen verkleinert werden, wobei dieselben Teile verwendet werden können. Das Vortriebselement 22 ist in eine gewünschte Startposition auf das Antriebselement 21 schraubbar. Es ergeben sich Abstufungen der unterschiedlichen Startpositionen für das Vortriebselement 22 durch dessen axial sich erstreckenden Verdrehsicherungsrippe 22b, welche in den korrespondierend hierzu angeordneten Verdrehsicherungsnute 13h des Mechanikhalters 13 geführt ist. Die Abstufung der unterschiedlichen Startpositionen des Vortriebselements 22 ist dabei weiter abhängig von der Ausgestaltung der Kupplung 23 bzw. der Federspule 20b bezüglich der Anzahl Haltenocken 23c, Kupplungsnocken distal 20e, Kupplungsnocken proximal 20p sowie von der Gewindesteigung und der Anzahl Gewindegänge an der aussenliegenden Gewindepartie 21a am Antriebselement 21. Zudem kann für kleinere Füllvolumen bzw. kleinere Hübe des Vortriebselements 22 ein angepasstes Spritzengehäuseteil 10b mit kürzeren Sichtfenstern 10c verwendet werden.

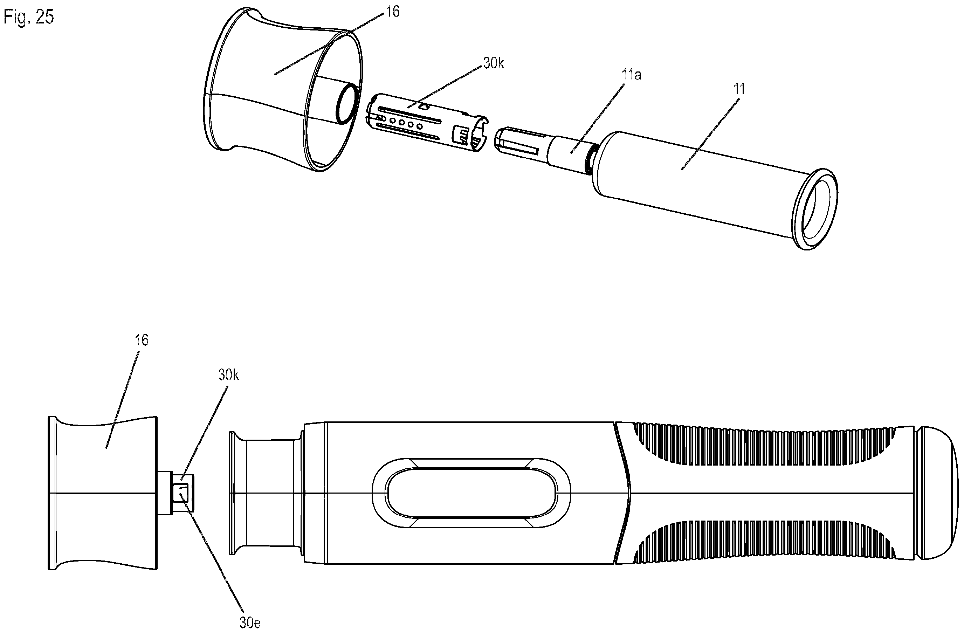

Wie in Fig. 25 gezeigt kann als Variante in der Gerätekappe 16 (anstelle des Insert 30) eine Abzughülse 30k mit distal und zur Achse L geneigten Krallen 301 formschlüssig, insbesondere irreversibel verschnappt, befestigt sein. Die Krallen 301 gleiten beim Fügen der Fertigspritze entlang der Mantelfläche der Nadelschutzkappe 11a. Beim Abziehen der Gerätekappe 16 greifen die Krallen in die Mantelfläche und nehmen die Nadelschutzkappe 11a mit, wodurch der Klemmbereich 11g und die Injektionsnadel 11b der Fertigspritze 11 freigelegt werden.

**BEZUGSZEICHENLISTE**

| **Zeichen** | **Teil** | **Alt. Kürzel** | **Anmerkung** |
|---|---|---|---|
| 1 | Autoinj ektor | | |
| | | | |
| 10a | Gehäuseteil, Antriebsgehäuseteil | DH | Drive Housing |
| 10b | Gehäuseteil, Spritzengehäuseteil | SHO | Syringe Housing |
| 10c | Ausnehmung, Sichtfenster | SHO-h | |
| 10d | Haltenocken | SHO-a | |
| 10e | Verdrehsicherungsrippen | SHO-b | |
| 10f | Doppelschnappnute | SHO-c | |
| 10g | Doppelhaltenocken | DH-a | |
| 10h | Verdrehsicherungsrippen | SHO-d | |
| 10i | Verdrehsicherungsflächen | DH-b | |
| 10k | Auflagefläche | SHO-e | |
| 10l | Auflagefläche | SHO-f | |
| 10m | Verdrehsicherungsnuten | SHO-g | |
| 10n | Sichtfensterrand | SHO-i | |
| 10o | Haltenocken | DH-d | |
| 10p | Auflagefläche | DH-e | |
| 10q | Verdrehsicherungsnute | DH-f | |
| 10r | Führungsrippe | DH-c | |
| 10s | Führungsrippe | DH-g | |
| 10t | Montagefenster | DH-i | |
| 10u | Rastnocke | DH-h | |
| 10v | Rastnute | SHO-j | |
| 10w | Endabschnitt | | |
| 10x | Spaltführung | | |
| 10y | Stufe | | |
| | | | |
| 11 | Fertigspritze | PFS | Pre Filled Syringe |
| 11a | Nadelschutzkappe | RNS | Rigid Needle Shield |
| 11b | Injektionsnadel | | |
| 11c | Zylinderförmiger Produktbehälter | | |
| 11d | Schulter, Auflagefläche | PFS-b | |
| 11e | Auflagefläche | PFS-c | |
| 11f | Schulter | PFS-a | an RNS |
| 11g | Klemmbereich | PFS-d | |
| 11i | Kontaktfläche | PFS-e | |
| 11j | Kontaktfläche | PFS-f | |
| 11h | Kontaktfläche | PFS-g | am Stopfen 26 |
| | | | |
| 12 | Spritzenhalter | SH | Syringe Holder |
| 12a | Sperrfläche, Halterippen | SH-f | |
| 12b | Führungshülse | | |
| 12c | Anschlag, Auflagekante | SH-e | |
| 12d | Ausnehmung | | nur bei drehend |
| 12e | Haltenocken | SH-a | |
| 12f | Halteschnapper | SH-b | |
| 12g | Auflageflächen | SH-c | |
| 12h | Verdrehsicherungsrippen | SH-d | |
| 12i | Verdrehsicherungsrippen | SH-g | |
| 12j | Sperrfläche | SH-h | |
| | | | |
| 13 | Mechanikhalter | MH | Mechanics Holder |
| 13a | Haltefederabschnitt; Spritzenadapter | SA | Syringe Adapter |
| 13b | Federhülse | | |
| 13c | Auflagefläche | SA-a | |
| 13d | flexible Elemente | SA-b | |
| 13e | Halteschnapper | MH-k | |
| 13f | Auflagefläche | MH-1 | |
| 13g | Verdrehsicherungsrippen | MH-m | |
| 13h | Verdrehsicherungsnuten | MH-a | |
| 13i | Halteschnapper | MH-b | |
| 13j | Halteschnapper | SA-c | |
| 13k | Halteöffnung | MH-c | |
| 13l | Auflagefläche | SA-d | |
| 13m | Auflagefläche | MH-d | |
| 13n | Verdrehsicherungsnute | SA-e | |
| 13o | Verdrehsicherungsrippe | MH-e | |
| 13p | Auflagefläche | MH-f | |
| 13r | Führungsfläche | MH-g | |
| 13s | Haltefläche | MH-h | |
| 13t | Führungsöffnung | MH-i | |
| 13q | Auflagefläche | MH-n | |
| 13u | Federaufnahme | MH-o | |
| 13v | Haltenocken | MH-s | |
| 13w | Kontaktfläche | MH-t | |
| 13x | Verdrehsicherungsrippe | MH-u | |
| 13y | Auflagefläche | MH-p | |
| 13z | Auflagefläche | MH-r | |
| | | | |
| 14 | Nadelschutzhülse | CS | Cover Sleeve |
| 14a | Arm | | |
| 14b | Abschnitt | | |
| 14c | Flansch | | |
| 14d | Halteschnapper | CS-b | |
| 14e | Verdrehsichungsnute | CS-a | |
| 14f | Halteöffnungen | CS-c | |
| 14g | Halteöffnungen | CS-d | |
| 14h | Verdrehsicherungsnuten | CS-e | |
| 14i | Kontaktfläche | CS-f | |
| 14j | Sperrfläche | CS-g | |
| 14k | Halteöffnung | CS-h | |
| | | | |
| 15 | Nadelschutzfeder | CSS | Cover Sleeve Spring |
| 15a | distales Federende | CSS-a | |
| 15b | proximales Federende | CSS-b | |
| | | | |
| 16 | Gerätekappe | CR | Cap Remover |
| 16a | Rippe, Verdrehsicherungsrippe | CR-b | |
| 16b | Schnappnocke, Haltenocken | CR-a | |
| 16c | Rastnocke | | nur bei drehend |
| | | | |
| 17 | Schalthülse | TOS | Telescopic Outer Sleeve |
| 17a | Auflagefläche | TOS-a | |
| 17b | Federauflage | TOS-b | |
| 17c | Führungsfläche | TOS-c | |
| 17d | Halteöffnung | TOS-d | |
| 17e | Sperrschnapper | TOS-j | |
| 17f | Haltefläche | TOS-i | |
| 17g | Kontaktfläche | TOS-h | |
| 17h | Sperrfläche | TOS-e | |
| 17i | Sperrfläche | TOS-f | |
| 17j | Montagenute | TOS-k | |
| | | | |
| 18 | Sperrhülse | TIS | Telescopic Inner Sleeve |
| 18a | Verriegelungsglied; Sperrarme | TIS-g | |
| 18b | Haltenocken | TIS-a | |
| 18c | Haltenocken | TIS-b | |
| 18d | Führungsfläche | TIS-c | |
| 18e | Kontaktfläche | TIS-d | |
| 18f | Sperrnocken | TIS-h | |
| 18g | Halteflächen | TIS-i | |
| 18h | Haltearm | TIS-f | |
| 18i | Kontaktfläche | TIS-e | |
| 18j | Auflagefläche | TIS-k | |
| 18k | Sperrfläche | TIS-1 | |
| | | | |
| 20a | Spiralfeder, Triebfeder | DSP | Drive Spring |
| 20b | Federspule | DS | |
| 20c | Auflagefläche | DS-a | |
| 20d | Verdrehsicherungsfläche | DS-b | |
| 20e | Kupplungsnocken distal | DS-e | |
| 20f | Kontaktfläche | DS-f | |
| 20g | Auflagefläche | DS-c | |
| 20h | äusseres Federende | DSP-b | |
| 20i | inneres Federende | DSP-a | |
| 20j | Federaufnahme | DS-d | |
| 20k | Kontaktfläche | DS-g | |
| 20l | Haltenocken | DS-h | |
| 20m | Verdrehsicherungsrippen | DS-i | |
| 20n | Auflagefläche | DS-k | |
| 20o | Kontaktfläche | DS-m | |
| 20p | Kupplungsnocken proximal | DS-1 | |
| | | | |
| 21 | Antriebselement | TR | Threaded Rod |
| 21a | aussenliegende Gewindepartie | TR-a | |
| 21b | umlaufende Schulter | TR-b | |
| 21c | proximale Auflagefläche | TR-c | |
| 21d | Verdrehsicherungsfläche | TR-d | |
| | | | |
| 22 | Vortriebselement | PR | Plunger Rod |
| 22a | innenliegende Gewindepartie | PR-a | |
| 22b | Verdrehsicherungsrippen | PR-b | |
| 22c | Auflagefläche | PR-c | |
| | | | |
| 23 | Kupplungshülse | CL | Clutch |
| 23a | Haltenocken, Kupplungsnocken | CL-e | |
| 23b | Federauflage | CL-a | |
| 23c | Haltenocken | CL-b | |
| 23d | Kontaktfläche | CL-c | |
| 23e | Haltearme | CL-d | |
| 23f | Kontaktfläche | CL-f | |
| 23g | Haltefläche | CL-g | |
| 23h | Auflagefläche | CL-h | |
| 23i | Schürze | | |
| | | | |
| 25a | Anzeigeelement | IR | Indicator Ring |
| 25b | Fenster, Anzeigefenster | IW | Indicator Window |
| 25c | Abschluss, Anzeigekappe | IC | Indicator Cap |
| 25d | Eingriffselement, Federarm | IR-d | |
| 25e | Auflagefläche | IR-a | |
| 25f | Haltenocken | IR-b | |
| 25g | Verdrehsicherungsnute | IR-c | |
| 25h | Halteöffnung | IW-a | |
| 25i | Auflagefläche | IW-f | |
| 25j | Verdrehsicherungsnute | IW-b | |
| 25k | Halteschnapper | IC-a | |
| 25l | Halteöffnung | IW-c | |
| 25m | Auflagefläche | IC-b | |
| 25n | Kontaktfläche | IW-g | |
| 25o | Auflagefläche | IW-d | |
| 25p | Kontrastmuster | IR-f | |
| 25q | Rastnocken | IR-e | |
| 25r | Rasterung | IW-e | |
| 25s | Füllprofil | | |
| | | | |
| 26 | Stopfen | PL | Plunger |
| | | | |
| 30 | Insert | CRI | Insert |
| 30a | Schnapparm mit Haken, Haltearm mit Haltenocken | CRI-d | inkl. CRI-g |
| 30b | Erste Schnappnut, distale Halteöffnung | CRI-e | |
| 30c | Zweite Schnappnut,proximale Halteöffnung | CRI-a | |
| 30d | Erste Verschnappung, distale Halteöffnung | CRI-h | |
| 30e | Zweite Verschnappung, proximale Halteöffnung | CRI-c | |
| 30f | Führungsnut | | Variante |
| 30g | Nut, Verdrehsicherungsnuten | CRI-b | |
| 30h | Stirnseite, Auflagefläche | CRI-i | |
| 30i | Montagenocken | | Variante |
| 30j | Halteschlitze | CRI-f | |
| 30k | Abzughülse | | Variante |
| 30l | Krallen | | Variante |
| | | | |
| 80 | Antriebsvorrichtung | DU | Drive Unit |
| | | | |
| 90 | Produktbehälteraufnahmevorrichtung | SU | Syringe Unit |
| | | | |
| L | Längsachse, Achse | | |

## Patentansprüche

1. Autoinj ektor (1) umfassend
- eine Antriebsvorrichtung (80) aufweisend ein erstes Gehäuseteil (10a) umfassend eine Antriebsbaugruppe (20, 21, 22),
- eine Produktbehälteraufnahmevorrichtung (90) aufweisend ein zweites Gehäuseteil (10b) definierend eine Längsachse (L), welche sich von distal nach proximal erstreckt, umfassend einen Spritzenhalter (12) ausgebildet zur Aufnahme einer vorgefüllten Fertigspritze (11) mit einem zylinderförmigem Produktbehälter (11c) und einem zur Ausschüttung eines Medikaments aus dem Produktbehälter (11c) entlang der Längsachse (L) durch die Antriebsbaugruppe (20, 21, 22) bewegbaren Stopfen (26), wobei das erste Gehäuseteil (10a) und das zweite Gehäuseteil (10b) koaxial in Fügerichtung verbindbar sind, indem an einem der beiden Gehäuseteile (10a, 10b) ein verbindungsseitiger Endabschnitt (10w) so abgestuft ist, dass dieser in das andere der beiden Gehäuseteile (10a, 10b) eingeschoben werden kann, wobei sich zwischen den beiden Gehäuseteilen (10a, 10b) im Bereich des Endabschnitts (10w) eine umlaufende Spaltführung (10x) ausbildet,
**dadurch gekennzeichnet, dass**
im Bereich des Endabschnitts (10w) eine erste Rastverbindung (10u, 10v) und eine zweite und dritte Rastverbindung (10f, 10g) vorgesehen sind, welche in Fügerichtung einzeln nacheinander einrastbar sind und wobei gegen die Fügerichtung die erste der Rastverbindungen (10u, 10v) lösbar ist und zumindest die dritte oder letzte Rastverbindung (10f, 10g) verriegelt.

2. Autoinj ektor (1) nach dem vorhergehenden Anspruch, **dadurch gekennzeichnet, dass** das erste Gehäuseteil (10a) und das zweite Gehäuseteil (10b) gegenseitig durch eine bezüglich der Längsachse (L) parallele Linearbewegung in Fügerichtung verbindbar oder gegen die Fügerichtung lösbar sind.

3. Autoinj ektor (1) nach einem der vorhergehenden Ansprüche, **dadurch gekennzeichnet, dass** durch die erste Rastverbindung (10u, 10v) und die zweite und dritte Rastverbindung (10f, 10g), eine erste, zweite und dritte Rastposition des ersten Gehäuseteil (10a) relativ zum zweiten Gehäuseteil (10b) entlang der Längsachse (L) definiert wird.

4. Autoinjektor (1) nach einem der vorhergehenden Ansprüche, **dadurch gekennzeichnet, dass** die erste, zweite und dritte Rastverbindung aus je am ersten und zweiten Gehäuseteil (10a, 10b) ausgebildeten Nocken und Nuten im Inneren der Spaltführung (10x) gebildet sind.

5. Autoinjektor (1) nach einem der vorhergehenden Ansprüche, **dadurch gekennzeichnet, dass** das eine der Gehäuseteile (10a, 10b) mit einer den Endabschnitt (10w) einleitenden radialen Stufe (10y) am verbindungsseitigen Ende des anderen Gehäuseteils (10a, 10b) umlaufend anstossbar ist, wobei die radiale Stufe (10y) in Umfangrichtung stetig gewellt oder unstetig abgesetzt sein kann.

6. Autoinjektor (1) nach dem vorhergehenden Anspruch, **dadurch gekennzeichnet, dass** das eine der Gehäuseteile (10a, 10b) mit der den Endabschnitt (10w) einleitenden radialen Stufe (10y) am verbindungsseitigen Ende des anderen Gehäuseteils (10a, 10b) umlaufend anstösst, wenn die dritte oder letzte der Rastverbindungen (10f, 10g) verriegelt ist.

7. Autoinjektor (1) nach einem der vorhergehenden Ansprüche, **dadurch gekennzeichnet, dass** die beiden Gehäuseteile (10a, 10b) im Bereich des Endabschnitts (10w) Längsführungen aufweisen und/oder einen nicht kreisförmigen Querschnitt der Spaltführung (10x) ausbilden.

8. Autoinjektor (1) nach einem der vorhergehenden Ansprüche, **dadurch gekennzeichnet, dass** das eine der Gehäuseteile (10a, 10b) an seinem Endabschnitt (10w) zumindest eine Montageöffnung (10t) aufweist, welche zumindest in der ersten Rastposition von Aussen zugänglich ist und in zumindest der dritten oder letzten Rastposition vom andern der Gehäuseteile (10a, 10b) vollständig überdeckt wird.

9. Autoinjektor (1) nach einem der vorhergehenden Ansprüche, **dadurch gekennzeichnet, dass** die beiden Gehäuseteile (10a, 10b) genau drei Rastpositionen entlang der Längsachse (L) einnehmen können.

10. Autoinjektor (1) nach einem der vorhergehenden Ansprüche, **dadurch gekennzeichnet, dass** die beiden Gehäuseteile (10a, 10b) in der dritten oder letzten Rastposition zusätzlich zur Rastverbindung (10f, 10g) durch eine Klebung oder Schweissung gegenseitig befestigt werden.

11. Autoinj ektor (1) nach einem der vorhergehenden Ansprüche, **dadurch gekennzeichnet, dass** dieser weiter einen Spritzenadapter (13a) aufweist, welcher mit seinem distalen Ende (13c) an das proximale Ende der Fertigspritze (11) anstossbar ist, wobei das proximale Ende des Spritzenadapters (13a) durch ein elastisches Element (13d) gebildet ist, welches mit dem ersten Gehäuseteil (10a) wirkverbunden ist.

12. Autoinjektor (1) nach dem vorhergehenden Anspruch, **dadurch gekennzeichnet, dass** das elastische Elemente (13d) des Spritzenadapters (13a), an das die Auflagefläche (13c) angebunden ist, durch die Bewegung in die dritte Rastposition gespannt wird.

13. Verfahren für die Endmontage eines Autoinjektors (1) welcher eine Längsachse (L) definiert, aufweisend die Schritte:
- Bereitstellen einer Produktbehälteraufnahmevorrichtung (90) aufweisend ein erstes Gehäuseteil (10a)
- Bereitstellen einer Antriebsvorrichtung (80) aufweisend ein zweites Gehäuseteil (10b)
- Fügen der Gehäuseteile (10a, 10b) in Fügerichtung in eine durch eine erste Rastverbindung (10u, 10v) definierte erste Rastposition
- Einsetzen eines Montagewerkzeugs durch eine Montageöffnung (10t) an einem der Gehäuseteile (10a, 10b) wenn diese in der ersten Rastposition sind
- Fügen der Gehäuseteile (10a, 10b) in Fügerichtung in eine zweite Rastposition welche durch eine zweite Rastverbindung (10f, 10g) der Gehäuseteile (10a, 10b) gehalten wird
- Entfernen des Montagewerkzeugs aus der Montageöffnung (10t) an einem der Gehäuseteile (10a, 10b) wenn diese in der zweiten Rastposition sind
- Fügen der Gehäuseteile (10a, 10b) in Fügerichtung in eine dritte Rastposition welche durch eine dritte Rastverbindung (10f, 10g) der Gehäuseteile (10a, 10b) gehalten und verriegelt wird
- Vollständiges Abdecken der Montageöffnung (10t) an einem der Gehäuseteile (10a, 10b) durch das andere der Gehäuseteile (10a, 10b) wenn diese in der dritten oder letzten Rastposition sind.

14. Verfahren für die Endmontage eines Autoinjektors (1) nach dem vorhergehenden Anspruch weiter aufweisend einen oder mehrere der folgenden Schritte:
- Einstellen einer Startposition für ein Vortriebselement (22) durch Aufschrauben auf ein Antriebselement (21)
- Positionieren der Produktbehälteraufnahmevorrichtung (90) und der Antriebsvorrichtung (80) koaxial der Längsachse (L) und Fügen der Gehäuseteile (10a, 10b) in Fügerichtung in eine Rastposition welche durch eine in und gegen die Fügerichtung lösbare Rastverbindung (10u, 10v) der Gehäuseteile (10a, 10b) gehalten wird
- Lagern und/oder Transportieren der Produktbehälteraufnahmevorrichtung (90) und der Antriebsvorrichtung (80) zusammen verbunden in der Rastposition der Gehäuseteile (10a, 10b)
- Bereitstellen einer Fertigspritze (11)
- Lösen der Rastverbindung (10u, 10v) der Gehäuseteile (10a, 10b) gegen die Fügerichtung und Separieren der Antriebsvorrichtung (80) von der Produktbehälteraufnahmevorrichtung (90)
- Einsetzen der Fertigspritze (11) koaxial oder achsparallel zur Längsachse (L) in die Produktbehälteraufnahmevorrichtung (90)
- Halten und/oder Positionieren eines im Innern des einen Gehäuseteile (10a, 10b) beweglichen Bauteils (17) mittels dem durch die Montageöffnung (10t) eingesetzten Montagewerkzeug.
- Spannen eines elastischen Spritzenadapters (13a) bei Fügen der Gehäuseteile (10a, 10b) von der zweiten in die dritte oder letzte Rastposition.

15. Verfahren für die Endmontage eines Autoinjektors (1) nach einem der zwei vorhergehenden Ansprüche ausgeführt an der Vorrichtung nach einem der Ansprüche 1 bis 12.
